# FASCICULE DE BREVET EUROPEEN

(11) **EP 1 699 778 B1**
(45) Date de publication et mention de la délivrance du brevet: **14.07.2010**
(21) Numéro de dépôt: 04805591.7
(22) Date de dépôt: 30.11.2004
(51) Int. Cl.: C07D 401/06, C07D 405/14, C07D 409/14, C07D 401/14, A61K 31/497, A61P 25/28

(54) **DERIVES DE (4-PHENYLPIPERAZIN-1-YL)ACYLPIPERIDINE, LEUR PREPARATION ET LEUR APPLICATION EN THERAPEUTIQUE**
(4-PHENYLPIPERAZIN-1-YL)ACYLPIPERIDINDERIVATE, DEREN HERSTELLUNG UND DEREN VERWENDUNG IN THERAPEUTKA
(4-PHENYLPIPERAZIN-1-YL)ACYLPIPERIDINE DERIVATIVES, PREPARATION THEREOF AND APPLICATION OF SAME IN THERAPEUTICS

(30) Priorité: 01.12.2003 FR 0314173
(43) Date de publication de la demande: 13.09.2006
(73) Titulaire: Sanofi-Aventis, 75013 Paris (FR)
(72) Inventeur: DOS SANTOS, Victor, F-34130 Valergues (FR); WAGNON, Jean, F-34070 Montpellier (FR)
(74) Mandataire: Kugel, Dominique
(86) Numéro de dépôt international: PCT/FR2004/003067
(87) Numéro de publication internationale: WO 2005/054227

(56) Documents cités:
- WO-A-00/69828
- WO-A-00/69829
- WO-A-01/49684
- WO-A-03/104225
- WO-A-03/104226

## Description

La présente invention a pour objet des dérivés de (4-phénylpipérazin-1-yl)acylpipéridine substitués, leur préparation et leur application en thérapeutique.

Les composés selon la présente invention présentent une affinité pour le récepteur p75^{NTR} des neurotrophines.

Les neurotrophines appartiennent à une famille de protéines possédant une structure et des fonctions proches et incluant le facteur de croissance nerveuse (NGF, de l'anglais Nerve Growth Factor), le BDNF, (de l'anglais Brain Derived Neurotrophic Factor), la neurotrophine-3 (NT-3, de l'anglais Neurotrophin-3), la neurotrophine-4/5 (NT-4/5, de l'anglais Neurotrophin-4/5) et la neurotrophine 6 (NT-6, de l'anglais Neurotrophin-6). Les effets biologiques de ces protéines (survie et différenciation) s'exercent par interaction avec des récepteurs membranaires à activité tyrosine kinase (trk-A, trk-B et trk-C) (H. THOENEN, Science, 1995, 270, 593-598 ; G.R. LEWIN et Y.A. BARDE, Annu. Rev. Neurosci., 1996, 19, 289-317 ; M.V. CHAO, J. Neurobiol., 1994, 25, 1373-1385 ; M. BOTHWELL, Annu. Rev. Neurosci., 1995, 18, 223-253 ; G. DECHANT et Y.A. BARDE, Curr. Opin. Neurobiol., 1997, 7, 413-418). Toutefois de nombreux travaux montrent le rôle prépondérant du récepteur p75^{NTR} dans l'activité des neurotrophines.

Le récepteur p75^{NTR}, récepteur de toutes les neurotrophines, est une glycoprotéine transmembranaire de la famille du récepteur du facteur de nécrose tumorale (TNF, de l'anglais Tumor Necrosis Factor) (W.J. FRIEDMAN et L.A. GREENE, Exp. Cell. Res., 1999, 253, 131-142 ; J. MELDOSIS et al., Trends Pharmacol. Sci., 2000, 21, 242-243). Plusieurs fonctions biologiques sont attribués au récepteur p75^{NTR} : d'une part, la modulation de l'affinité des neurotrophines pour les récepteurs trk ; d'autre part, en l'absence de trk, une induction d'un signal de mort cellulaire par apoptose qui s'effectue par homodimérisation du récepteur et activation de la voie des céramides.

L'apoptose ou mort cellulaire programmée est un mécanisme physiologique d'élimination des cellules dans de nombreux tissus. En particulier, l'apoptose joue un rôle prépondérant dans l'embryogénèse, la morphogénèse et le renouvellement cellulaire. L'apoptose est un phénomène génétiquement contrôlé qui n'intervient qu'à un stade avancé et irréversible de lésion cellulaire.

De nombreux travaux montrent que l'apoptose intervient dans plusieurs pathologies du système nerveux central comme la sclérose latérale amyotrophique, la sclérose en plaques, les maladies d'Alzheimer, de Parkinson et d' Huntington et les maladies à prion. De plus, la mort neuronale par apoptose intervient également très précocement après une ischémie cérébrale et cardiaque. La mort cellulaire est également un phénomène prépondérant dans l'athérosclérose, en effet on évalue à 80% les zones de nécrose dans les lésions primaires d'athérosclérose chez l'homme (M.L. BOCHATON-PIALAT et al., Am. J. Pathol., 1995, 146, 1-6 ; H. PERLMAN, Circulation, 1997, 95, 981-987). L'apoptose est également impliquée dans les mécanismes conduisant à la mort cellulaire consécutive à une ischémie-reperfusion cardiaque (H. YAOITA et al., Cardiovasc. Res., 2000, 45, 630-641).

Plusieurs travaux montrent que le signal pro-apoptotique p75^{NTR} dépendant est observé dans différents types cellulaires dont les cellules neuronales, les oligodendrocytes, les cellules de Schwann et aussi les cellules hépatiques, cardiaques et musculaires lisses (J.M. FRADE et al., Nature, 1996, 383, 166-168 ; P. LASACCIA-BONNEFIL et al., Nature, 1996, 383, 716-719 ; M. SOILU-HANNINEN et al., J. Neurosci., 1999, 19, 4828-4838 ; N. TRIM et al., Am. J. Pathol., 2000, 156, 1235-1243 ; S.Y. WANG et al., Am. J. Pathol., 2000, 157, 1247-1258). De plus, plusieurs expériences réalisées in vivo montrent une augmentation de l'expression de p75^{NTR} après ischémie dans des régions du cerveau et du coeur dans lesquelles une apoptose massive est enregistrée. Ces résultats suggèrent donc que p75^{NTR} peut jouer un rôle prépondérant dans les mécanismes conduisant à la mort neuronale par apoptose post ischémie (P.P. ROUX et al., J. Neurosci., 1999, 19, 6887-6896 ; J.A. PARK et al., J. Neurosci., 2000, 20, 9096-9103).

Le récepteur p75^{NTR} est décrit comme cible cellulaire du peptide Prion (V. DELLA-BIANCA et al., J. Biol. Chem., 2001, in Press) et du peptide β-Amyloide (S. RABIZADEH et al., Proc. Natl. Acad. Sci. USA, 1994, 91, 10703-10706) et serait ainsi impliqué dans les phénomènes d'apoptose induits par ces composés. Ces résultats supportent l'hypothèse selon laquelle p75^{NTR} jouerait un rôle important dans la mort neuronale induite par la protéine prion infectieuse (encéphalopathie spongiforme transmissible) ou par la protéine beta Amyloide (maladie d'Alzheimer).

Des études récentes suggèrent que le récepteur p75^{NTR} jouerait également un rôle important dans la régénération axonale via sa fonction de co-récepteur du récepteur Nogo (WONG et al., Nature Neurosci., 2002, 5, 1302-1308 ; Kerracher and Winton, Neuron, 2002, 36, 345-348). En effet, plusieurs protéines associées à la myéline (myelin-associated glycoprotein, MAG, Nogo-A et oligodendrocyte myelin glycoprotein OMgp) inhibent la régénération nerveuse au niveau central lors d'un traumatisme médullaire ou crânien. Ces protéines sont localisées dans la membrane des oligodendrocytes directement adjacents à l'axone et inhibent la croissance neuritique en se liant avec une forte affinité au récepteur Nogo localisé sur la membrane axonale. Le récepteur p75^{NTR} est associé au récepteur Nogo et impliqué dans la signalisation des effets inhibiteurs de ces protéines de la myéline vis-à-vis de la croissance axonale. De ce fait, le récepteur p75^{NTR} joue un rôle majeur dans la régulation de la plasticité neuronale et dans les intéractions neurones-glie et représente ainsi une cible thérapeutique de choix pour promouvoir la régénération nerveuse.

Au niveau périphérique, des travaux récents montrent une augmentation de l'expression de p75^{NTR} et des neurotrophines et une apoptose massive dans des lésions d'athérosclérose. De plus, un effet pro-aiagiogène et vasodilateur du NGF est également enregistré. Enfin, une nouvelle forme de p75^{NTR} tronquée dans la partie extracellulaire a été mise en évidence ainsi que son rôle majeur dans la vasculogénèse établie (D. VON SHACK et al., Nature Neuroscience, 2001, 4, 977-978). L'ensemble de ces données récentes suggèrent que p75^{NTR} sous forme entière ou tronquée pourrait également jouer un rôle prépondérant dans les pathologies vasculaires.

Un certain nombre de composés sont connus pour interagir avec le système trkAlNGF/p75^{NTR} ou pour posséder une activité de type NGF. Ainsi la demande de brevet WO 00/59893 décrit des dérivés de pyrimidines substituées qui démontrent une activité de type NGF et/ou qui augmentent l'activité du NGF sur les cellules PC12. Les demandes de brevet WO 00/69828 et WO 00/69829 décrivent des composés polycycliques qui inhibent la liaison du NGF au récepteur p75^{NTR} dans des cellules qui n'expriment pas le récepteur trkA. La demande WO 94/11373 décrit des dérivés de pyridazinoquinazolone qui se lient au récepteur p75^{NTR} des neurotrophines. La demande WO 94/22866 décrit des dérivés de pyrazoloquinazolone qui se lient au NGF de manière spécifique afin d'éviter sa fixation au récepteur p75^{NTK} mais lui permettant d'interagir avec le récepteur trk. La demande WO 01/49684 décrit des dérivés de tétrahydropyridines substitués qui possèdent une activité vis-à-vis de la modulation du TNF-alpha.

La demande WO03/104225 décrit des dérivés de pipérazinylacylpipéridine qui présentent une affinité pour le récepteur p75^{NTR} des neurotrophines. La demande WO 03/104226 décrit des dérivés de pipérazinylarylpipéridine qui présentent une affinité pour le récepteur p75^{NTR} des neurotrophines.

On a maintenant trouvé de nouveaux dérivés de (4-phénylpipérazin-1-yl) acylpipéridine qui présentent une affinité pour le récepteur p75^{NTR}.

La présente invention a pour objet des composés répondant à la formule (I) : 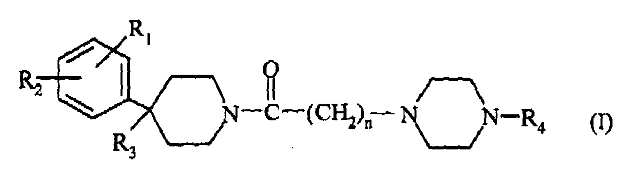 dans laquelle:
- n est 1 ou 2;
- R₁ représente un atome d'halogène ; un radical trifluorométhyle ; un (C₁-C₄)alkyle ; un (C₁-C₄)alcoxy ; un radical trifluorométhoxy ;
- R₂ représente un atome d'hydrogène ou un atome d'halogène ;
- R₃ représente un atome d'hydrogène ; un groupe -OR₅ ; un groupe -CH₂OR₅ ; un groupe -NR₆R₇ ; un groupe NR₈COR₉ ; un groupe -CH₂NR₁₀R₁₁ ; un (C₁-C₄)alcoxycarbonyle ; un groupe -CONR₁₂R₁₃ ; un radical -CN ;
- R₄ représente un phényle non substitué, mono-, di- ou trisubstitué par un substituant choisi indépendamment parmi un atome d'halogène, un (C₁-C₄)alkyle, un (C₁-C₄)alcoxy, un radical trifluorométhyle ;
- R₅ représente un atome d'hydrogène ou un (C₁-C₄)alkyle ;
- R₆ et R₇ représentent chacun indépendamment un atome d'hydrogène ou un (C₁-C₄)alkyle ;
- R₈ représente un atome d'hydrogène ou un (C₁-C₄)alkyle ;
- R₉ représente un (C₁-C₄)alkyle ;
- R₁₀ et R₁₁ représentent chacun indépendamment un atome d'hydrogène ou un (C₁-C₄)alkyle ; R₁₁ peut de plus représenter un groupe -CH₂R₁₄ ;
- ou bien R₁₀ et R₁₁ ensemble avec l'atome d'azote auquel ils sont liés constituent un hétérocycle choisi parmi l'aziridine, l'azétidine, la pyrrolidine, la pipéridine ou la morpholine ;
- R₁₂ et R₁₃ représente chacun indépendamment un atome d'hydrogène ou un (C₁-C₄)alkyle ;
- ou bien R₁₂ et R₁₃ ensemble avec l'atome d'azote auquel ils sont liés constituent un hétérocycle choisi parmi l'azétidine, la pyrrolidine, la pipéridine, la morpholine ou la pipérazine non substituée ou substituée en position -4- par un (C₁-C₄)alkyle ;
- R₁₄ représente un groupe aromatique choisi parmi : 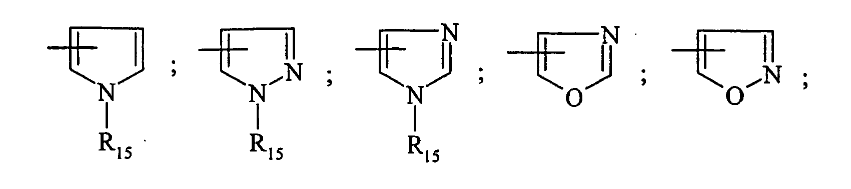 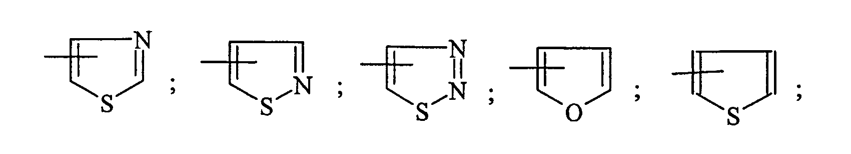 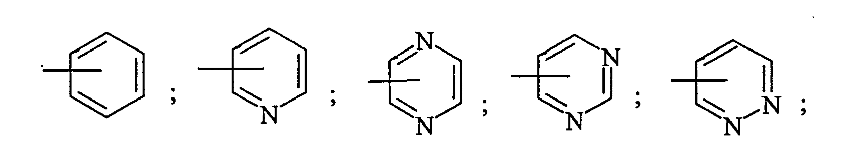 lesdits groupes aromatiques étant non substitués, mono- ou disubstitués par un substituant choisi indépendamment parmi un atome d'halogène, un (C₁-C₃)alkyle ou un (C₁-C₃)alcoxy ;
- R₁₅ représente un atome d'hydrogène ou un (C₁-C₃)alkyle.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables mais les sels d'autres acides utiles pour la purification ou l'isolement des composés de formule (I) sont également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Par atome d'halogène on entend un atome de brome, de chlore, de fluor ou d'iode.

Par (C₁-C₃)alkyle ou respectivement (C₁-C₄)alkyle on entend un radical alkyle linéaire ou ramifié de un à trois atomes de carbone ou respectivement de un à quatre atomes de carbone tel que le radical méthyle, éthyle, propyle, isopropyle, butyle, isobutyle, sec-butyle, *tert*-butyle.

Par (C₁-C₄)alcoxy on entend un radical alcoxy linéaire ou ramifié de un à quatre atomes de carbone, tel que le radical méthoxy, éthoxy, propoxy, isopropoxy, butoxy, isobutoxy, sec-butoxy ou *tert*-butoxy.

Parmi les composés de formule (I) objets de l'invention, on peut citer les composés préférés qui se définissent comme suit :
- n est 1 ou 2 ;
- et/ou R₁ est en position -3- du phényle et représente un radical trifluorométhyle ;
- et/ou R₂ représente un atome d'hydrogène ;
- et/ou R₃ représente un hydroxy, un aminométhyle, un (2-furylméthyl amino)méthyle, un (2-thiénylméthylamino)méthyle, un (2-pyridinylméthyl amino)méthyle, un (3-pyridinylméthylamino)méthyle, un (4-pyridinylméthyl amino)méthyle, un (méthylamino)méthyle, un (diméthylamino)méthyle, un (N-méthyléthylamino)méthyle, un (diéthylamino)méthyle, un azétidin-1-ylcarbonyle ; un aminocarbonyle ; un (N-méthyl-2-furylméthylamino)méthyle ; un (3-furylméthylamino)méthyle ;
- et/ou R₄ représente un phényle, un 4-chlorophényle, un 3-chlorophényle, un 4-fluorophényle, un 2,4-dichlorophényle, un 3,4-dichlorophényle, un 3,5-dichlorophényle, un 4-méthylphényle, un 2,4-diméthylphényle, un 3,4-diméthylphényle, un 3-méthoxyphényle, un 4-méthoxyphényle, un 2,4-diméthoxyphényle, un 3,4-diméthoxyphényle, un 3-(trifluorométhyl)phényle ; un 2,3-diméthylphényle.

Parmi ces derniers composés préférés, on préfère particulièrement les composés de formule (I) pour lesquels :
- n est 1 ou 2 ;
- R₁ est en position -3- du phényle et représente un radical trifluorométhyle ;
- R₂ représente un atome d'hydrogène ;
- R₃ représente un hydroxy, un aminométhyle, un (2-furylméthyl amino)méthyle, un (2-thiénylméthylamino)méthyle, un (2-pyridinylméthylamino)méthyle, un (3-pyridinylméthylamino)méthyle, un (4-pyridinylméthylamino)méthyle, un (méthylamino)méthyle, un (diméthylamino)méthyle, un (N-méthyléthylamino)méthyle, un (diéthylamino)méthyle, un azétidin-1-ylcarbonyle ; un aminocarbonyle ; un (N-méthyl-2-furylméthylamino)méthyle ; un (3-furylméthylamino)méthyle ;
- R₄ représente un phényle, un 4-chlorophényle, un 3-chlorophényle, un 4-fluorophényle, un 2,4-dichlorophényle, un 3,4-dichlorophényle, un 3,5-dichlorophényle, un 4-méthylphényle, un 2,4-diméthylphényle, un 3,4-diméthylphényle, un 3-méthoxyphényle, un 4-méthoxyphényle, un 2,4-diméthoxyphényle, un 3,4-diméthoxyphényle, un 3-(trifluorométhyl)phényle, un 2,3-diméthylphényle ;
à l'état de base ou de sel d'addition à un acide, ainsi qu'à l'état d'hydrate ou de solvat.

Parmi les composés de formule (I) objets de l'invention, on peut notamment citer les composés suivants :
- 4-[3-(trifluorométhyl)phényl]-1-[[4-[3-(trifluorométhyl)phényl]pipérazin-1-yl] acétyl]pipéridin-4-ol ;
- 1-[[4-(3,4-diméthylphényl)pipérazin-1-yl]acétyl]-4-[3-(trifluorométhyl)phényl] pipéridin-4-ol ;
- 1-[[4-(3,5-dichlorophényl)pipérazin-1-yl]acétyl]-4-[3-(trifluorométhyl)phényl] pipéridin-4-ol ;
- 1-[[4-(4-méthylphényl)pipérazin-1-yl]acétyl]-4-[3-(trifluorométhyl)phényl] pipéridin-4-ol ;
- [[1-[(4-phénylpipérazin-1-yl)acétyl]-4-[3-(trifluorométhyl)phényl]pipéridin-4-yl] méthyl]amine ;
- (2-furylméthyl)[[1-[(4-phénylpipérazin-1-yl)acétyl]-4-[3-(trifluorométhyl)phényl] pipéridin-4-yl]méthyl]amine ;
- [[1-[(4-phénylpipérazin-1-yl)acétyl]-4-[3-(trifluorométhyl)phényl]pipéridin-4-yl] méthyl](2-thiénylméthyl)amine ;
- [[1-[(4-phénylpipérazin-1-yl)acétyl]-4-[3-(trifluorométhyl)phényl]pipéridin-4-yl] méthyl](pyridin-2-ylméthyl)amine ;
- [[1-[(4-phénylpipérazin-1-yl)acétyl]-4-[3-(trifluorométhyl)phényl]pipéridin-4-yl] méthyl](pyridin-3-ylméthyl)amine ;
- [[1-[(4-phénylpipérazin-1-yl)acétyl]-4-[3-(trifluorométhyl)phényl]pipéridin-4-yl] méthyl](pyridin-4-ylméthyl)amine ;
- N-méthyl-1-[1-[(4-phénylpipérazin-1-yl)acétyl]-4-[3-(trifluorométhyl)phényl] pipéridin-4-yl]méthanamine ;
- N,N-diméthyl-1-[1-[(4-phénylpipérazin-1-yl)acétyl]-4-[3-(trifluorométhyl)phényl] pipéridin-4-yl]méthanamine ;
- N-méthyl-N-[[1-[(4-phénylpipérazin-1-yl)acétyl]-4-[3-(trifluorométhyl)phényl] pipéridin-4-yl]méthyl]éthanamine ;
- [[1-[[4-(4-fluorophényl)pipérazin-1-yl]acétyl]-4-[3-(trifluorométhyl)phényl] pipéridin-4-yl]méthyl]amine ;
- [[1-[[4-(3-méthoxyphényl)pipérazin-1-yl]acétyl]-4-[3-(trifluorométhyl)phényl] pipéridin-4-yl]méthyl]amine ;
- [[1-[[4-(3,4-dichlorophényl)pipérazin-1-yl]acétyl]-4-[3-(trifluorométhyl)phényl] pipéridin-4-yl]méthyl]amine ;
- [[1-[[4-(2,4-diméthylphényl)pipérazin-1-yl]acétyl]-4-[3-(trifluorométhyl)phényl] pipéridin-4-yl]méthyl]méthylamine ;
- [[1-[[4-(2,4-diméthylphényl)pipérazin-1-yl]acétyl]-4-[3-(trifluorométhyl)phényl] pipéridin-4-yl]méthyl]diméthylamine ;
- [[1-[[4-(3,4-diméthoxyphényl)pipérazin-1-yl]acétyl]-4-[3-(trifluorométhyl)phényl] pipéridin-4-yl]méthyl]amine ;
- [[1-[[4-(3,4-diméthoxyphényl)pipérazin-1-yl]acétyl]-4-[3-(trifluorométhyl)phényl] pipéridin-4-yl]méthyl]diméthylamine ;
- N-éthyl-N-[[1-[[4-(3-méthoxyphényl)pipérazin-1-yl]acétyl]-4-[3-(trifluorométhyl) phényl] pipéridin-4-yl]méthyl]éthanamine ;
- 1-[2-[4-(azétidin-1-ylcarbonyl)-4-[3-(trifluorométhyl)phényl]pipéridin-1-yl]-2-oxoéthyl]-4-phénylpipérazine ;
- [[1-[[4-(3,4-diméthoxyphényl)pipérazin-1-yl]acétyl]-4-[3-(trifluorométhyl)phényl] pipéridin-4-yl]méthyl]méthylamine ;
- 1-[[4-(4-chlorophényl)pipérazin-1-yl]acétyl]-4-[3-(trifluorométhyl)phényl] pipéridin-4-ol ;
- 1-[[4-(3-chlorophényl)pipérazin-1-yl]acétyl]-4-[3-(trifluorométhyl)phényl] pipéridin-4-ol ;
- 1-[[4-(4-méthoxyphényl)pipérazin-1-yl]acétyl]-4-[3-(trifluorométhyl)phényl] pipéridin-4-ol ;
- 1-[[4-(3-méthoxyphényl)pipérazin-1-yl]acétyl]-4-[3-(trifluorométhyl)phényl] pipéridin-4-ol ;
- 1-[(4-phénylpipérazin-1-yl)acétyl]-4-[3-(trifluorométhyl)phényl]pipéridine-4-carboxamide ;
- 1-[[4-(2,4-diméthylphényl)pipérazin-1-yl]acétyl]-4-[3-(trifluorométhyl)phényl] pipéridine-4-carboxamide ;
- 1-[[4-(2,4-diméthoxyphényl)pipérazin-1-yl]acétyl]-4-[3-(trifluorométhyl)phényl] pipéridine-4-carboxamide ;
- 1-[[4-(2,4-dichlorophényl)pipérazin-1-yl]acétyl]-4-[3-(trifluorométhyl)phényl] pipéridine-4-carboxamide ;
- 1-[3-(4-phénylpipérazin-1-yl)propanoyl]-4-[3-(trifluorométhyl)phényl]pipéridin-4-ol;
- 1-[3-[4-(4-méthylphényl)pipérazin-1-yl]propanoyl]-4-[3-(trifluorométhyl)phényl] pipéridin-4-ol ;
- 1-[3-[4-(4-fluorophényl)pipérazin-1-yl]propanoyl]-4-[3-(trifluorométhyl)phényl] pipéridin-4-ol ;
- 1-[3-[4-(4-méthoxyphényl)pipérazin-1-yl]propanoyl]-4-[3-(trifluorométhyl)phényl] pipéridin-4-ol ;
- [[1-[[4-(3,4-diméthoxyphényl)pipérazin-1-yl]acétyl]-4-[3-(trifluorométhyl)phényl] pipéridin-4-yl]méthyl](2-furylméthyl)méthylamine ;
- (3-furylméthyl)[[1-[(4-phénylpipérazin-1-yl)acétyl]-4-[3-(trifluorométhyl)phényl] pipéridin-4-yl]méthyl]amine ;
- [[1-[[4-(2,3-diméthylphényl)pipérazin-1-yl]acétyl]-4-[3-(trifluorométhyl)phényl] pipéridin-4-yl]méthyl]amine ;
à l'état de base ou de sel d'addition à un acide, ainsi qu'à l'état d'hydrate ou de solvat.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans "Protective Groupe in Organic Synthesis", Green et al., 2nd Edition (John Wiley & Sons, Inc., New York).

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans "Advances in Organic Chemistry", J. March, 3rd Edition, Wiley Interscience, p. 310-316.

Conformément à l'invention, on peut préparer les composés de formule (I) dans laquelle n = 1, selon un procédé **caractérisé en ce que** :
a1) on fait réagir un composé de formule : 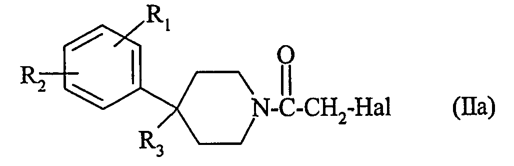 dans laquelle R₁, R₂ et R₃ sont tels que définis pour un composé de formule (I) et Hal représente un atome d'halogène, le chlore ou le brome de préférence, étant entendu que lorsque R₃ contient une fonction hydroxyle ou amine, ces fonctions peuvent être protégées, avec un composé de formule : 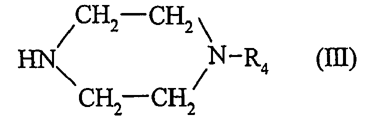 dans laquelle R₄ est tel que défini pour un composé de formule (I) ;
b1) et, après déprotection éventuelle des fonctions hydroxyle ou amine contenues dans R₃, on obtient le composé de formule (I).

Eventuellement, on transforme le composé de formule (I) en l'un de ses sels d'addition à un acide.

Conformément à l'invention, on peut préparer les composés de formule (I) dans laquelle n = 2, selon un procédé **caractérisé en ce que :**
a2) on fait réagir un composé de formule : 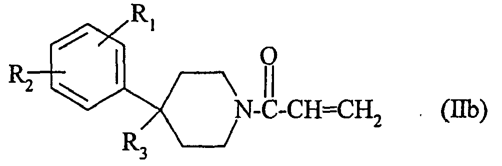 dans laquelle R₁, R₂ et R₃ sont tels que définis pour un composé de formule (I), étant entendu que lorsque R₃ contient une fonction hydroxyle ou amine, ces fonctions peuvent être protégées, avec un composé de formule : 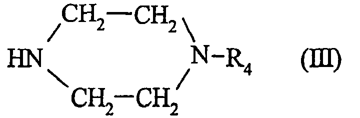 dans laquelle R₄ est tel que défini pour un composé de formule (I) ;
b2) et, après déprotection éventuelle des fonctions hydroxyle ou amine contenues dans R₃, on obtient le composé de formule (I).

Eventuellement, on transforme le composé de formule (I) en l'un de ses sels d'addition à un acide.

A l'étape a1) ou à l'étape a2), lorsqu'on fait réagir un composé de formule (IIa) ou (IIb) avec un composé de formule (III), la réaction s'effectue en présence d'une base choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium et en l'absence ou en présence d'un iodure de métal alcalin tel que l'iodure de potassium ou l'iodure de sodium. La réaction s'effectue dans un solvant tel que l'acétonitrile, le N,N-diméthylformamide, le toluène ou le propan-2-ol et à une température comprise entre la température ambiante et la température de reflux du solvant.

Eventuellement, à l'étape b1) ou à l'étape b2), la déprotection des fonctions hydroxyle ou amine contenues dans R₃, s'effectue selon les méthodes classiques bien connues de l'homme de l'art.

Selon une variante du procédé et lorsque R₃ représente un groupe -CH₂NR₁₀R₁₁ dans lequel R₁₀ et R₁₁ représentent chacun l'hydrogène :
a3) on fait réagir un composé de formule : 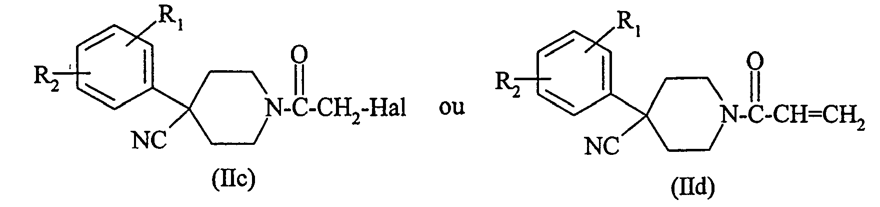 dans laquelle R₁ et R₂ sont tels que définis pour un composé de formule (I), et Hal représente un atome d'halogène, de préférence le chlore ou le brome, avec un composé de formule : 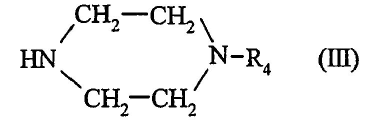 dans laquelle R₄ est tel que défini pour un composé de formule (I), pour obtenir un composé de formule : 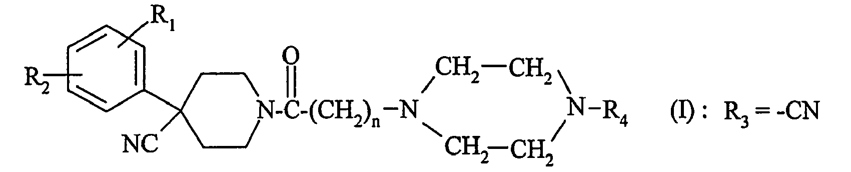
b3) on réduit le groupe cyano du composé de formule (I) pour obtenir un composé de formule (I) dans laquelle R₃ = CH₂NH₂.

Eventuellement, on transforme le composé de formule (I) en l'un de ses sels d'addition à un acide.

A l'étape a3), la réaction entre le composé de formule (IIc) ou (IId) et le composé de formule (III) s'effectue comme précédemment décrit à l'étape a1) ou a2) du procédé selon l'invention.

A l'étape b3), la réduction du groupe cyano du composé de formule (I) dans laquelle R₃ = -CN s'effectue selon les méthodes classiques. Ainsi, par exemple, la réduction s'effectue par hydrogénation, en présence d'un catalyseur tel que le nickel de Raney^{®} ou le rhodium sur alumine, et en présence ou en l'absence d'ammoniaque, dans un solvant tel que le méthanol, le N,N-diméthylformamide ou le tétrahydrofurane ou un mélange de ces solvants et à une température comprise entre la température ambiante et 60°C.

Selon une autre variante du procédé et lorsque R₃ représente un groupe -CH₂NR₁₀R₁₁ dans lequel R₁₁ représente un groupe -CH₂R₁₄ :
a 4) on fait réagir un composé de formule : 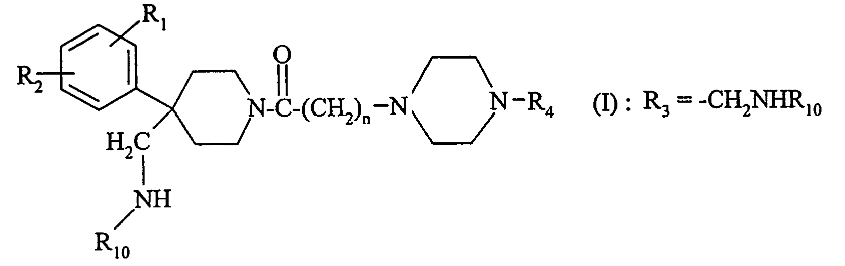 dans laquelle R₁, R₂, R₄, R₁₀ et n sont tels que définis pour un composé de formule (I), avec un composé de formule : 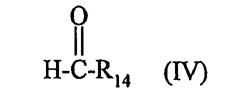 dans laquelle R₁₄ est tel que défini pour un composé de formule (I), en présence d'un acide, dans un solvant, puis on réduit le sel d'iminium formé intermédiairement au moyen d'un agent réducteur.

Eventuellement, on transforme le composé de formule (I) en l'un de ses sels d'addition à un acide.

La réaction s'effectue en présence d'un acide tel que l'acide acétique, dans un solvant tel que le dichlorométhane ou le tétrahydrofurane, à une température comprise entre la température ambiante et la température de reflux du solvant, et forme *in-situ* une imine intermédiaire qui est réduite chimiquement en utilisant, par exemple, le cyanoborohydrure de sodium ou le triacétoxyborohydrure de sodium ou catalytiquement en utilisant l'hydrogène et un catalyseur tel que le palladium sur charbon ou le nickel de Raney^{®}.

Un composé de formule (I) dans laquelle R₃ représente un groupe -CH₂NR₁₀R₁₁ dans lequel R₁₀ = H et R₁₁ = (C₁-C₄)allcyle peut également être préparé par réaction d'un composé de formule (I) dans laquelle R₃ = -CH₂NH₂ avec un halogénure de (C₁-C₄)alkyle, en présence d'une base telle qu'un carbonate de métal alcalin comme le carbonate de potassium dans un solvant tel que l'acétonitrile, le N,N-diméthylformamide ou le tétrahydrofurane et à une température comprise entre la température ambiante et la température de reflux du solvant. Par une réaction identique on prépare les composés de formule (I) dans laquelle R₁₀ et R₁₁ représentent chacun un (C₁-C₄)alkyle semblable ou différent.

Un composé de formule (I) dans laquelle R₃ représente un groupe -CH₂NR₁₀R₁₁ dans lequel R₁₀ = H ou (C₁-C₄)alkyle et R₁₁ = (C₁-C₄)alkyle peut également être préparé par réaction d'un composé de formule (I) dans laquelle R₃ = - CH₂-NHR₁₀ avec le formaldéhyde ou avec un aldéhyde de formule OHC-(C₁-C₃)alkyle ou avec une cétone correspondante, en présence d'un agent réducteur tel que le borohydrure de sodium ou le triacétoxyborohydrure de sodium et en présence d'un acide tel que l'acide acétique, dans un solvant tel que le dichlorométhane ou le tétrahydrofurane et à une température comprise entre 0°C et la température ambiante.

Un composé de formule (I) dans laquelle R₃ représente un groupe -CH₂NR₁₀R₁₁ dans lequel R₁₀ et R₁₁ ensemble avec l'atome d'azote auquel ils sont liés constituent l'aziridine peut également se préparer par cyclisation d'un composé intermédiaire correspondant dans lequel R₃ représente un groupe -CH₂NH-CH₂CH₂-Cl, en présence d'une base telle qu'un carbonate de métal alcalin comme le carbonate de potassium, et en présence d'un iodure alcalin tel que l'iodure de potassium, dans un solvant comme l'acétonitrile et à une température comprise entre la température ambiante et la température de reflux du solvant ; le composé intermédiaire correspondant se prépare par réaction d'un composé de formule (I) dans laquelle R₃ = -CH₂NH₂ avec du chloroacétaldéhyde selon la méthode précédemment décrite.

Un composé de formule (I) dans laquelle R₃ représente un groupe -CH₂NR₁₀R₁₁ dans lequel R₁₀ et R₁₁ ensemble avec l'atome d'azote auquel ils sont liés constituent l'azétidine, la pyrrolidine, la pipéridine ou respectivement la morpholine peut également se préparer par réaction d'un composé de formule (I) dans laquelle R₃ = -CH₂NH₂ avec un composé de formule Hal-(CH₂)₃-Hal, Hal-(CH₂)₄-Hal, Hal-(CH₂)₅-Hal ou, respectivement, Hal-CH₂CH₂-O-CH₂CH₂-Hal, dans laquelle Hal représente un atome d'halogène, de préférence le chlore ou le brome, en présence d'une base telle qu'un carbonate de métal alcalin comme le carbonate de potassium et en présence d'un iodure alcalin tel que l'iodure de potassium, dans un solvant comme l'acétonitrile, l'éthylène glycol ou un mélange de ces solvants et à une température comprise entre la température ambiante et la température de reflux du solvant.

Un composé de formule (I) dans laquelle R₃ représente un groupe -CONR₁₂R₁₃ peut également se préparer par réaction d'un composé intermédiaire correspondant dans lequel R₃ représente un carboxy avec un composé de formule HNR₁₂R₁₃ selon les méthodes classiques du couplage peptidique ; le composé intermédiaire correspondant se prépare selon les méthodes classiques par traitement acide ou basique d'un composé de formule (I) dans laquelle R₃ représente un (C₁-C₄) alcoxycarbonyle ou par réaction d'un composé de formule (I) dans laquelle R₃ = -CN avec une base forte comme un hydroxyde de métal alcalin tel que l'hydroxyde de potassium, dans un solvant tel que le toluène ou l'éthylène glycol à une température comprise entre la température ambiante et la température de reflux du solvant.

Un composé de formule (I) dans laquelle R₃ représente un groupe -CH₂OR₅ dans lequel R₅ représente un atome d'hydrogène peut également se préparer par traitement acide ou basique d'un composé intermédiaire correspondant dans lequel R₃ représente un groupe -CH₂OCO(C₁-C₄)alkyle.

Les composés de formule (I) ainsi obtenus peuvent être ultérieurement séparés du milieu réactionnel et purifiés selon les méthodes classiques, par exemple par cristallisation ou chromatographie.

Les composés de formule (I) ainsi obtenus sont isolés sous forme de base libre ou de sel, selon les techniques classiques.

Les composés de formule (IIa) se préparent par réaction d'un dérivé de pipéridine de formule : 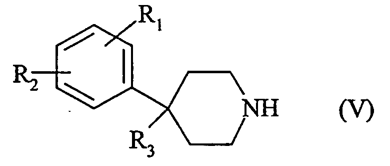 dans laquelle R₁, R₂ et R₃ sont tels que définis pour un composé de formule (I), avec un composé de formule : 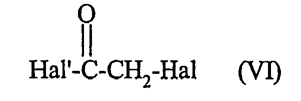 dans laquelle Hal et Hal' représentent chacun indépendamment un atome d'halogène, de préférence le chlore ou le brome. La réaction s'effectue en présence d'une base telle que la triéthylamine, la N,N-diisopropyléthylamine ou la N-méthylmorpholine, dans un solvant tel que le dichlorométhane, le chloroforme, le tétrahydrofurane, le dioxane ou un mélange de ces solvants et à une température comprise entre 0°C et la température ambiante.

Les composés de formule (IIb) se préparent par réaction du composé de formule (V) avec un composé de formule : 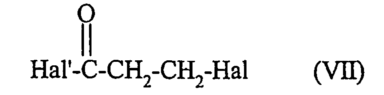 dans laquelle Hal et Hal' sont tels que définis ci-dessus, dans les conditions opératoires ci-dessus mentionnées.

De même, on prépare les composés de formule (IIc) ou respectivement (IId) par réaction d'un composé de formule : 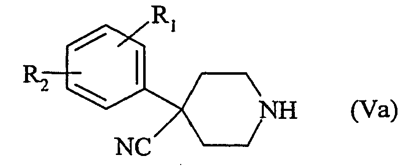 dans laquelle R₁ et R₂ sont tels que définis pour un composé de formule (I), avec un composé de formule (VI) ou respectivement (VII) selon les mêmes conditions opératoires que ci-dessus.

Les composés de formule (VI) ou (VII) sont commerciaux, connus ou se préparent selon des méthodes connues.

Les composés de formule (III) sont commerciaux ou préparés selon des méthodes connues telles que celles décrites dans Tetrahedron Lett., 1996, 37 (47), 8487-8488 ; Tetrahedron Lett., 1997, 38 (39), 6875-6876 ; Tetrahedron Lett., 2000, 41 (16), 2881-2884 ; Syn. Lett., 2000, 5, 674-676 ; Bioorg. Med. Chem. Lett., 2000, 10, 2489-2491 ; Bioorg. Med. Chem., 2002, 10, 4023-4027.

Les composés de formule (IV) sont disponibles dans le commerce ou décrits dans la littérature, ou bien peuvent être préparés selon des méthodes qui y sont décrites telles que dans J. Org. Chem., 1961, 26, 2976 ; Chim. Ind. (Milan), 1968, 50, 264 ; J. Med. Chem., 1970, 13, 1208-1212 ; J. Heterocycl. Chem., 1990, 27 (1), 1-12 ; Synth. Commun., 1995, 25 (9), 1383-1390 ; Bioorg. Med. Chem. Lett., 2003, 13 (3), 463-466.

Les composés de formule (V) sont commerciaux, connus ou se préparent selon des méthodes connues telles que celles décrites dans EP-0 474 561, EP-0 673 928 ou WO 96/23787.

Les composés de formule (V) sont généralement préparés sous forme protégée sur l'atome d'azote de la pipéridine ; après une étape de déprotection, on obtient les composés de formule (V) eux-mêmes.

Particulièrement, on prépare un composé de formule (V) dans laquelle R₃ représente un groupe -OR₅ dans lequel R₅ = H par réaction d'un dérivé organomagnésien de formule : 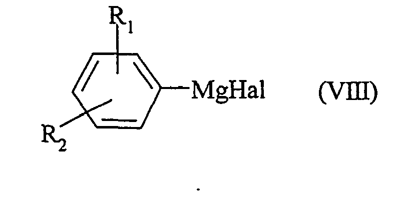 dans laquelle R₁ et R₂ sont tels que définis pour un composé de formule (I) et Hal représente un atome d'halogène, le brome de préférence, avec la 1-benzyl-4-pipéridinone, dans un solvant tel que l'éther diéthylique ou le tétrahydrofurane, à une température comprise entre la température ambiante et la température de reflux du solvant.

Les dérivés organomagnésiens de formule (VIII) se préparent selon les méthodes classiques bien connues de l'homme de l'art à partir des dérivés halogénés correspondants.

A partir des composés de formule (V) dans laquelle R₃ = -OH on prépare les composés de formule (V) dans laquelle R₃ = -OR₅ dans lequel R₅ représente un (C₁-C₄)alkyle par réaction d'alkylation selon les méthodes connues de l'homme de l'art.

Les composés de formule (V) dans laquelle R₃ = -OH et qui portent un groupe protecteur sur l'atome d'azote de la pipéridine, peuvent subir une réaction de Ritter par action de l'acétonitrile, en milieu acide, pour préparer les composés de formule (V) dans laquelle R₃ = -NHCOCH₃, selon la méthode décrite dans EP-0 474 561. Par hydrolyse en milieu acide fort, on prépare ensuite les composés de formule (V) dans laquelle R₃ = -NR₆R₇ dans lequel R₆ = R₇ = H. Selon les méthodes décrites dans EP-0 673 928 ou WO 96/23787, on prépare les composés de formule (V) dans laquelle R₃ = -NR₆R₇ dans lequel R₆ et/ou R₇ représente un (C₁-C₄)alkyle.

Les composés de formule (V) dans laquelle R₃ = -NR₈COR₉ dans lequel R₉ est un (C₁-C₄)alkyle, ou bien R₃ = -CH₂NR₁₀R₁₁ dans lequel R₁₀ et R₁₁ représentent chacun indépendamment un hydrogène ou un (C₁-C₄)alkyle, ou bien R₃ = (C₁-C₄) alcoxycarbonyle, ou bien R₃ = -CONR₁₂R₁₃ se préparent selon les méthodes décrites dans WO 96/23787.

Un composé de formule (V) dans laquelle R₃ = -CH₂NR₁₀R₁₁ dans lequel R₁₀ = R₁₁ = H se prépare à partir d'un composé de formule (Va) selon la méthode précédemment décrite pour un composé de formule (I).

Un composé de formule (V) dans laquelle R₃ = -CH₂NR₁₀R₁₁ dans lequel R₁₀ = H ou (C₁-C₄)alkyle et R₁₁ = (C₁-C₄)alkyle ou un groupe -CH₂R_{14,} se prépare selon la méthode précédemment décrite pour un composé de formule (I).

Un composé de formule (V) dans laquelle R₃ = -CH₂NR₁₀R₁₁ dans lequel R₁₀ = H et R₁₁ = -CH₃ peut également se préparer par réduction d'un composé intermédiaire correspondant dans lequel R₃ = -CH₂NHCHO au moyen d'un agent réducteur tel que l'hydrure d'aluminium et de lithium, dans un solvant tel que l'éther ou le tétrahydrofurane et à une température comprise entre la température ambiante et la température de reflux du solvant. Le composé intermédiaire correspondant se prépare par réaction d'un composé de formule (V) dans laquelle R₃ = -CH₂NH₂ avec le formiate d'éthyle, à une température comprise entre la température ambiante et 60°C.

Un composé de formule (V) dans laquelle R₃ = -CH₂NR₁₀R₁₁ dans lequel R₁₀ et R₁₁ ensemble avec l'atome d'azote auquel ils sont liés constituent l'aziridine, l'azétidine, la pyrrolidine, la pipéridine ou la morpholine se prépare selon les méthodes précédemment décrites pour un composé de formule (I).

Un composé de formule (V) dans laquelle R₃ = -CONR₁₂R₁₃ dans lequel R₁₂ = R₁₃ = H peut aussi se préparer par réaction d'un composé de formule (Va), protégé sur l'atome d'azote de la pipéridine, avec le peroxyde d'hydrogène, en présence d'une base forte comme un hydroxyde de métal alcalin tel que l'hydroxyde de sodium et d'un catalyseur de transfert de phase tel qu'un sel d'ammonium quaternaire substitué, le chlorure de trioctylméthylammonium par exemple, dans un solvant tel que le toluène en mélange avec de l'eau, à une température comprise entre la température ambiante et la température de reflux du solvant.

Les composés de formule (Va) se préparent selon des méthodes connues telles que celles décrites dans Bioorg. Med. Chem. Lett., 1999, 9, 3273-3276 et dans J. Med. Chem., 1999, 42 (23), 4778-4793.

A partir des composés de formule (V) dans laquelle R₃ = -CH₂OH on prépare les composés de formule (V) dans laquelle R₃ = -CH₂OR₅ dans lequel R₅ représente un (C₁-C₄)alkyle par réaction d'alkylation selon les méthodes connues de l'homme de l'art.

Les composés de formule (V) dans laquelle R₃ = -CH₂OR₅ dans lequel R₅ représente un atome d'hydrogène se préparent par réduction d'un composé de formule (V) dans laquelle R₃ représente un méthoxycarbonyle selon les méthodes connues de l'homme de l'art.

Les composés de formule (V) dans laquelle R₃ représente un (C₁-C₄) alcoxycarbonyle se préparent par réaction d'estérification d'un composé intermédiaire correspondant dans lequel R₃ représente un carboxy selon les méthodes connues de l'homme de l'art ; le composé intermédiaire correspondant se prépare par réaction d'un composé de formule (Va) avec une base forte comme un hydroxyde de métal alcalin tel que l'hydroxyde de potassium, dans un solvant tel que le toluène ou l'éthylène glycol à une température comprise entre la température ambiante et la température de reflux du solvant.

Les groupes N-protecteurs éventuellement utilisés sont les groupes N-protecteurs classiques bien connus de l'homme de l'art tels que par exemple le groupe *tert-*butoxycarbonyle; fluorénylméthoxycarbonyle, benzyle, benzhydrylidène ou benzyloxycarbonyle.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le TABLEAU I ci-après, qui illustre les structures chimiques et les propriétés physiques de quelques composés selon l'invention.

Dans les Préparations et dans les Exemples on utilise les abréviations suivantes :
éther : éther diéthylique
éther iso : éther diisopropylique
DMSO : diméthylsulfoxyde
DMF : N,N-diméthylformamide
THF : tétrahydrofurane
DCM : dichlorométhane
AcOEt : acétate d'éthyle
DIPEA : diisopropyléthylamine
TFA : acide trifluoroacétique
BOP : benzotriazol-1-yloxytris(diméthylamino)phosphoniumhexafluoro phosphate
PyBOP : benzotriazol-1-yloxytripyrrolidinophosphonium hexafluorophosphate
Ether chlorhydrique 2N : solution 2N d'acide chlorhydrique dans l'éther diéthylique
F : point de fusion
TA : température ambiante
Eb : température d'ébullition
CLHP : chromatographie liquide haute performance
Silice H : gel de silice 60 H commercialisé par Merck (DARMSTAD)
Solution tampon pH = 2 : solution de 16,66 g de KHSO₄ et 32,32 g de K₂SO₄ dans 1 litre d'eau.

Les spectres de résonance magnétique du proton (RMN ¹H) sont enregistrés à 200 MHz dans du DMSO-d₆, en utilisant le pic du DMSO-d₆ comme référence. Les déplacements chimiques δ sont exprimés en parties par million (ppm). Les signaux observés sont exprimés ainsi : s: singulet ; se : singulet élargi ; d : doublet ; d.d :
doublet dédoublé ; t : triplet ; td : triplet dédoublé ; q : quadruplet ; m : massif ; mt : multiplet.

Les spectres RMN confirment les structures des composés.

Les composés selon l'invention sont analysés par couplage LC/UV/MS (chromatographie liquide/détection UV/spectrométrie de masse).

Pour les composés on vérifie que leur spectre de masse obtenus en mode Electrospray positif (ESI+) sont compatibles avec la masse molaire calculée.

Les spectres de masse des composés selon l'invention présentent, en général, comme pic de base l'ion moléculaire MH⁺.

### PREPARATIONS

### 1. Préparations des composés de formules (V) et (Va).

### Préparation 1.1

### Chlorhydrate de 4-[3-(trifluorométhyl)phényl]-4-pipéridinol.

(V), HCl: R₁ = 3-CF₃ ; R₂ = H ; R₃ = -OH.

### A) Chlorhydrate de 1-benzyl-4-[3-(trifluorométhyl)phényl]-4-pipéridinol.

On chauffe à 30°C un mélange de 180 g de magnésium dans 2670 ml de THF, ajoute 33 ml d'une solution de 1670 g de 1-bromo-3-(trifluorométhyl)benzène dans 1330 ml de THF, puis lentement le reste de la solution de manière à atteindre puis à maintenir le reflux du THF, et laisse 2 heures à reflux sous agitation. On ajoute ensuite lentement une solution de 1000 g de 1-benzyl-4-pipéridinone dans 3200 ml de THF et chauffe à reflux pendant 2 heures. Après refroidissement à TA, on verse le mélange réactionnel, en 30 minutes, sur une solution de 1870 g de chlorure d'ammonium dans 6700 ml d'eau et laisse 2 heures sous agitation à 20-25°C. Après décantation, on lave la phase organique par 5330 ml d'eau et évapore le solvant sous vide. On reprend le résidu dans 5330 ml d'éther, ajoute lentement une solution de 210 g d'HCl gaz dans 800 ml de propan-2-ol en maintenant la température inférieure à 25°C, laisse 40 minutes sous agitation et essore les cristaux formés. On reprend les cristaux dans 2000 ml d'éther et essore à nouveau. On obtient 1080 g du produit attendu après recristallisation dans le mélange propan-2-ol/EtOH (70/30 ; v/v).

### B) Chlorhydrate de 4-[3-(trifluorométhyl)phényl]-4-pipéridinol.

On hydrogène, à 50°C et sous 2 bars de pression, un mélange de 1000 g du composé obtenu à l'étape précédente et 83 g de palladium sur charbon à 10 % (50 % d'humidité) dans 2910 ml d'EtOH et 2910 ml de MeOH. On filtre le catalyseur, le lave deux fois par 660 ml de MeOH et concentre sous vide le filtrat et les jus de lavages. On reprend le résidu dans 3320 ml d'éther et laisse 1 heure 30 minutes sous agitation à TA. On essore le précipité formé, le lave par 280 ml d'éther et le sèche sous vide à 40°C. On obtient 726 g du produit attendu.

### Préparation 1.2

### Chlorhydrate de 4-[3-(trifluorométhyl)phényl]-4-pipéridinecarbonitrile.

(Va), HCl : R₁ = 3-CF₃ ; R₂ = H.

### A) 2-(2,2-diéthoxyéthyl)-4,4-diéthoxy-2-[3-(trifluorométhyl)phényl]butanenitrile.

On laisse 5 minutes à TA sous agitation un mélange de 30 g de 3-(trifluorométhyl)phénylacétonitrile et 14,4 g d'amidure de sodium dans 400 ml de toluène, ajoute 66 ml de bromoacétaldéhyde diéthylacétal puis chauffe à 60°C pendant 3 heures. On concentre sous vide, reprend le résidu à l'eau, extrait à l'éther, sèche la phase organique sur Na₂SO₄ et évapore le solvant sous vide. On chromatographie le résidu sur gel de silice H en éluant par le mélange DCM/AcOEt (100/5 ; v/v). On obtient 26 g du produit attendu.

### B) 4-Oxo-2-(2-oxoéthyl)-2-[3-(trifluorométhyl)phényl]butanenitrile.

On laisse 1 heure sous agitation à 50°C un mélange de 23,9 g du composé obtenu à l'étape précédente dans 90 ml d'acide formique. On ajoute de l'eau au mélange réactionnel, extrait à l'AcOEt, lave la phase organique à l'eau, par une solution de NaHCO₃ à 10 %, sèche sur Na₂SO₄ et évapore le solvant sous vide. On obtient 16 g du produit attendu que l'on utilise immédiatement à l'étape suivante.

### C) Chlorhydrate de 1-benzyl-4-[3-(trifluorométhyl)phényl]-4-pipéridinecarbonitrile.

On laisse une nuit sous agitation à TA un mélange de 16 g du composé obtenu à l'étape précédente, 6,25 ml de benzylamine, 48,6 g de triacétoxyborohydrure de sodium et 5 gouttes d'acide acétique dans 150 ml de DCM. On ajoute ensuite, goutte à goutte, 40 ml de MeOH puis chauffe à 60°C pendant 1 heure. On concentre sous vide le mélange réactionnel, extrait le résidu à l'AcOEt, lave la phase organique par une solution de NaHCO₃ à 10 %, à l'eau, sèche sur Na₂SO₄ et évapore le solvant sous vide. On reprend le résidu dans une solution saturée d'HCl gaz dans l'éther et essore le précipité formé. On obtient 18 g du produit attendu.

### D) Chlorhydrate de 4-[3-(trifluorométhyl)phényl]-4-pipéridinecarbonitrile.

On hydrogène pendant 3 heures, à TA et sous pression atmosphérique, un mélange de 2 g du composé obtenu à l'étape précédente et 0,2 g de palladium sur charbon à 10 % dans 30 ml de MeOH. On filtre le catalyseur sur Célite^{®} et concentre sous vide le filtrat. On obtient 1,5 g du produit attendu.

On peut également préparer ce composé en suivant les trois étapes ci-après :

### A') Bis(2-chloroéthyl)carbamate de tert-butyle.

A un mélange de 106 g de chlorhydrate de N,N-bis-(2-chloroéthyl)amine et 130 g de di-*tert*-butyldicarbonate dans 1500 ml de DCM, on ajoute, en goutte à goutte, à TA et en 1 heure 30 minutes, 83 ml de triéthylamine, puis laisse une nuit sous agitation à TA. On lave le mélange réactionnel à l'eau, sèche la phase organique sur Na₂SO₄ et évapore sous vide. On obtient 150 g du produit attendu que l'on utilise tel quel.

### B') 4-Cyano-4-[3-(trifluorométhyl)phényl]-1-pipéridine carboxylate de tert-butyle.

A une suspension de 56 g d'hydrure de sodium à 60 % dans l'huile dans 750 ml de DMSO et 250 ml de THF, on ajoute, en goutte à goutte, sous atmosphère inerte et à TA, une solution de 120 g de 3-(trifluorométhyl)phénylacétonitrile dans 250 ml de DMSO, puis, lentement, une solution de 150 g du composé obtenu à l'étape précédente dans 250 ml de DMSO et chauffe à 60°C pendant une nuit. On verse le mélange réactionnel dans un mélange glace/H₂O, extrait à l'éther, lave la phase organique à l'eau, par une solution saturée de NaCl, sèche sur Na₂SO₄ et évapore le solvant sous vide. On chromatographie le résidu sur gel de silice en éluant au DCM puis par le mélange DCM/AcOEt (80/20 ; v/v). On obtient 191 g du produit attendu qui cristallise, F = 72-73°C.

### C') Chlorhydrate de 4-[3-(trifluorométhyl)phényl]-4-pipéridinecarbonitrile.

On laisse 4 heures sous agitation à TA un mélange de 115 g du composé obtenu à l'étape précédente, 500 ml d'une solution d'HCl 2N dans l'éther et 150 ml de MeOH. On essore le produit cristallisé formé et le sèche. On obtient 75 g du produit attendu, F = 259°C.

### Préparation 1.3

### [4-[3-(trifluorométhyl)phényl]=4-pipéridinyl]méthylcarbamate de tert-butyle.

(V) : R₁ = 3-CF₃ ; R₂ = H ; R₃ = -CH₂NH-COOC(CH₃)₃.

### A) [1-Benzyl-4-[3-(trifluorométhyl)phényl]-4-pipéridinyl]méthylamine.

On hydrogène pendant une nuit, à TA et à pression atmosphérique, un mélange de 1,5 g du composé obtenu à l'étape C de la Préparation 1.2, 0,15 g de Nickel de Raney^{®} et 5 ml d'ammoniaque dans 20 ml de MeOH. On filtre le catalyseur et concentre sous vide le filtrat. On obtient 1,45 g du produit attendu.

### B) [1-Benzyl-4-[3-(trifluorométhyl)phényl]-4-pipéridinyl]méthylcarbamate de tert-butyle.

On chauffe à 40°C un mélange de 1,45 g du composé obtenu à l'étape précédente dans 20 ml d'AcOEt, ajoute 0,9 g de di-*tert*-butyldicarbonate puis chauffe à reflux pendant 30 minutes. Après refroidissement à TA, on ajoute de l'eau, extrait à l'AcOEt, sèche la phase organique sur Na₂SO₄ et évapore le solvant sous vide. On obtient 1,86 g du produit attendu.

### C) [4-[3-(trifluorométhyl)phënyl]-4-pipéridinyl]méthylcarbamate de tert-butyle.

On hydrogène pendant une nuit, à TA et à pression atmosphérique, un mélange de 1,8 g du composé obtenu à l'étape précédente et 0,18 g de palladium sur charbon à 10 % dans 20 ml de MeOH. On filtre le catalyseur et concentre sous vide le filtrat. On obtient 1,3 g du produit attendu sous forme d'huile.

### Préparation 1.4

### [[4-[3-(trifluorométhyl)phényl]pipéridin-4-yl]méthyl]carbamate de tert-butyle méthyle ;

(V) : R₁ = 3-CF₃ ; R₂ = H ; R₃ = -CH₂N(CH₃)-COOC(CH₃)₃.

### A) N,N-bis-(2-chloroéthyl)benzylamine.

On refroidit au bain de glace un mélange de 150 g de chlorhydrate de N,N-bis-(2-chloroéthyl)amine et 100 ml de bromure de benzyle dans 1000m de DMF, puis ajoute en goutte à goutte, 12ml de triéthylamine et laisse une nuit sous agitation à TA. On concentre sous vide le mélange réactionnel, reprend le résidu à l'eau, extrait 3 fois à l'éther, sèche les phases organiques sur Na₂SO₄ et évapore le solvant sous vide. On obtient 113 g du produit attendu.

### B) Chlorhydrate de 1-benzyl-4-[3-(trifluorométhyl)phényl]-4-pipéridinecarbonitrile.

A une suspension de 23,24 g d'hydrure de sodium à 60 % dans l'huile dans 100 ml de DMSO et 100 ml de THF, on ajoute, en goutte à goutte, sous atmosphère inerte et à TA, une solution de 50 g de 3-(trifluorométhyl)phénylacétonitrile dans 150 ml de DMSO et laisse 15 minutes sous agitation. On ajoute ensuite, en 1 heure, une solution de 62,43 g du composé obtenu à l'étape précédente dans 150ml de DMSO et laisse une nuit sous agitation à TA. On ajoute un mélange glace/eau, extrait à l'éther, sèche la phase organique sur Na₂SO₄ et évapore le solvant sous vide. On reprend le résidu dans 1000 ml d'EtOH chaud, laisse 48 heures sous agitation à TA et essore le produit cristallisé formé. On obtient 50 g du produit attendu.

### C) [1-Benzyl-4-[3-(trifluorométhyl)phényl]-4-pipéridinyl]méthylamine.

On dissout 30 g du composé obtenu à l'étape précédente dans une solution de NaOH à 10 %, extrait à l'éther, sèche la phase organique sur Na₂SO₄ et évapore le solvant sous vide. On reprend le produit sous forme de base libre dans 500 ml de MeOH et 30ml d'une solution d'ammoniaque à 20 %, on ajoute 3 g de nickel de Raney® et hydrogène pendant une nuit à TA et à pression atmosphérique. On filtre le catalyseur et concentre sous vide le filtrat. On reprend le résidu à l'eau, extrait à l'AcOEt, sèche la phase organique sur Na₂SO₄ et évapore le solvant sous-vide. On obtient 27 g du produit attendu.

### D) [[1-Benzyl-4-[3-(trifluorométhyl)phényl]-4-pipéridinyl]méthyl]formamide.

On laisse une nuit sous agitation à TA un mélange de 27 g du composé obtenu à l'étape précédente et 300 ml de formiate d'éthyle, puis chauffe à 60°C pendant 6 heures et laisse 48 heures sous agitation à TA. On concentre sous vide, reprend le résidu par une solution d'HCl à 10 %, lave la phase aqueuse acide à l'éther, ajoute de la glace et alcalinise par ajout d'une solution de NaOH à 10%, extrait à l'éther, sèche la phase organique sur Na₂SO₄ et évapore le solvant sous vide. On chromatographie le résidu sur gel de silice H en éluant au DCM puis par le mélange DCM/MeOH (100/4 ; v/v). On obtient 20 g du produit attendu.

### E) [[1-Benzyl-4-[3-(trifluorométhyl)phényl]-4-pipéridinyl]méthyl]méthylamine.

A une suspension de 4 g d'hydrure de lithium et d'aluminium dans 400 ml d'éther, on ajoute à TA 20 g du composé obtenu à l'étape précédente puis laisse 16 heures sous agitation à TA. On ajoute ensuite successivement 3 ml d'eau, 3 ml de NaOH à 30 % et 1 ml d'eau et laisse sous agitation. On filtre les sels minéraux sur Célite, décante le filtrat, sèche la phase organique sur Na₂SO₄ et évapore le solvant sous vide. On obtient 18 g du produit attendu.

### F) [[1-Benzyl-4-[3-(trifluorométhyl)phényl]-4-pipéridinyl]méthyl]méthylcarbamate de tert-butyle.

On laisse 1 heure sous agitation à TA un mélange de 18 g du composé obtenu à l'étape précédente et 9,6 g de di-*tert*-butyldicarbonate dans 300ml de DCM. On ajoute de l'eau au mélange réactionnel, extrait au DCM, sèche la phase organique sur Na₂SO₄ et évapore le solvant sous vide. On chromatographie le résidu sur gel de silice H en éluant par le mélange DCM/MeOH (100/2 ; v/v). On obtient 21g du produit attendu.

### G) [4-[3-(trifluorométhyl)phényl]pipéridin-4-yl]méthyl]carbamate de tert-butyle méthyle.

On hydrogène pendant 12 heures, à TA et à pression atmosphérique, un mélange de 21 g du composé obtenu à l'étape précédente et 2 g de palladium sur charbon à 10 % dans 300 ml de MeOH. On filtre le catalyseur et concentre sous vide le filtrat. On obtient 16 g du produit attendu.

### Préparation 1.5

### Dichlorhydrate de N,N-diméthyl-1-[4-[3-(trifluorométhyl)phényl]pipéridin-4-yl] méthanamine.

(V), 2HCl : R₁ = 3-CF₃ ; R₂ = H ; R₃ = -CH₂N(CH₃)₂.

### A) 4-(Aminométhyl)-4-[3-(trifluorométhyl)phényl]pipéridine-1-carboxylate de tert-butyle.

On hydrogène pendant 2 jours, à 25°C et sous pression atmosphérique, un mélange de 18,2 g du composé obtenu à l'étape B' de la Préparation 1.2, 1,8 g de nickel de Raney^{®} et 30 ml d'ammoniaque dans 600 ml de MeOH. On filtre le catalyseur et concentre sous vide le filtrat. On obtient 18 g du produit attendu que l'on utilise tel quel.

### B) 4-[(Diméthylamino)méthyl]-4-[3-(trifluorométhyl)phényl]pipéridine-1-carboxylate de tert-butyle.

A un mélange de 18 g du composé obtenu à l'étape précédente, 9,4 ml d'une solution de formaldéhyde à 37 % dans l'eau, 1 ml d'acide acétique dans 500 ml de THF, on ajoute, à TA, 106 g de triacétoxyborohydrure de sodium et laisse une nuit sous agitation à TA. On ajoute 100 ml de MeOH et chauffe à 70°C pendant 1 heure 30 minutes. On concentre sous vide, reprend le résidu à l'eau, alcalinise par ajout de NaOH concentrée, extrait au DCM, lave la phase organique à l'eau, sèche sur Na₂SO₄ et évapore le solvant sous vide. On chromatographie le résidu sur gel de silice en éluant par le mélange DCM/MeOH (96/4 ; v/v). On obtient 15,4 g du produit attendu sous forme d'huile qui se solidifie.

### C) Dichlorhydrate de NN-diméthyl-1-[4-[3-(trifluorométhyl)phényl]pipéridin-4-yl] méthanamine.

On laisse 3 heures sous agitation à TA un mélange de 15,4 g du composé obtenu à l'étape précédente, 150 ml d'une solution d'éther chlorhydrique 2N et 0,5 ml d'HCl concentré dans 20 ml de MeOH. On concentre sous vide, reprend le résidu dans l'éther, le triture et essore le précipité formé. On obtient 11,9 g du produit attendu, F = 240-245°C.

### Préparation 1.6

(V), HCl : R₁ = 3-CF₃ ; R₂ = H ; R₃ = -COOCH₃.

### A) Acide 1-benzyl-4-[3-(trifluorométhyl)phényl]-4-pipéridinecarboxylique.

On chauffe à reflux pendant 3 heures un mélange de 5 g du composé obtenu à l'étape C de la Préparation 1.2 et 4,25 g de KOH en pastilles dans 80 ml d'éthylène glycol. Après refroidissement à TA, on ajoute 100 ml d'eau, acidifie à pH = 6,5 par ajout d'une solution d'HCl à 10 %, essore le précipité formé et le sèche sous vide. On obtient 3,9 g du produit attendu, F = 243°C.

### B) 1-Benzyl-4-[3-(trifluorométhyl)phényl]-4-pipéridinecarboxylate de méthyle, chlorhydrate.

On chauffe à 60°C pendant 3 heures un mélange de 3 g du composé obtenu à l'étape précédente et 50 ml de chlorure de thionyle dans 100 ml de DCM. On concentre sous vide, reprend le résidu dans 100 ml de MeOH et chauffe une nuit à 60°C. On concentre sous vide et obtient 4 g du produit attendu, F = 230°C. C) 4-[3-(trifluorométhyl)phényl]-4-pipéridinecarboxylate de méthyle, chlorhydrate.

On hydrogène pendant une nuit, à TA et sous pression atmosphérique, un mélange de 4 g du composé obtenu à l'étape précédente et 0,4 g de palladium sur charbon à 10 % dans 200 ml de MeOH. On filtre le catalyseur et concentre sous vide le filtrat. On obtient 2,5 g du produit attendu.

### Préparation 1.7

### 4-(Azétidin-1-ylcarbonyl)-4-[3-(trifluorométhyl)phényl]pipéridine.

(V) : R₁ = 3-CF₃ ; R₂ = H ;

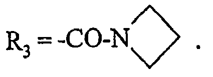

### A) Chlorhydrate de 4-(chloroformyl)-4-[3-(trifluorométhyl)phényl]pipéridine.

On chauffe à 60°C pendant 2 heures un mélange de 1 g du composé obtenu à l'étape A de la préparation 1.6 et 10 ml de chlorure de thionyle dans 10 ml de DCM. On concentre sous vide et obtient 1,05g du produit attendu que l'on utilise tel quel.

### B) 4-(Azétidin-1-ylcarbonyl)-1-benzyl-4-[3-(trifluorométhyl)phényl]pipéridine

On laisse une nuit sous agitation à TA un mélange de 1,05 g du composé obtenu à l'étape précédente, 0,283 g d'azétidine et 1,15 ml de triéthylamine dans 10 ml de DCM. On ajoute une solution saturée de K₂CO₃ au mélange réactionnel, extrait au DCM, sèche sur Na₂SO₄ et évapore sous vide le solvant. On chromatographie le résidu sur gel de silice en éluant par le mélange DCM/MeOH (97/3 ; v/v). On obtient 0,43 g du produit attendu.

### C) 4-(Azétidin-1-ylcarbonyl)-4-[3-(trifluorométhyl)phényl]pipéridine.

On hydrogène pendant une nuit, à 25°C et sous pression atmosphérique, un mélange de 0,43 g du composé obtenu à l'étape précédente, 1 g de palladium sur charbon à 10 % et 20 ml de MeOH. On filtre le catalyseur et concentre sous vide le filtrat. On obtient 0,33 g du produit attendu que l'on utilise tel quel.

### Préparation 1.8

### 4-[3-(trifluorométhyl)phényl]-4-pipéridinecarboxamide.

(V) : R₁ = 3-CF₃ ; R₂ = H ; R₃ = -CONH₂.

### A) 1-Benzyl-4-[3-(trifluorométhyl)phényl]-4-pipéridinecarboxamide.

On chauffe pendant 48 heures à 100°C un mélange de 5 g du composé obtenu à l'étape C de la Préparation 1.2, 30 ml de toluène, 30 ml d'une solution à 30 % d'H₂O₂, 30 ml d'une solution à 30 % de NaOH et 0,5 g d'aliquat 336 (chlorure de trioctylméthylammonium). On concentre sous vide, reprend le résidu à l'eau, extrait au DCM, sèche la phase organique sur Na₂SO₄ et évapore le solvant sous vide. On chromatographie le résidu sur gel de silice H en éluant par le mélange DCM/MeOH (100/3 ; v/v). On obtient 2,5 g du produit attendu. B) 4-[3-(trifluorométhyl)phényl]-4-pipéridinecarboxamide.

On hydrogène pendant 48 heures, à TA et sous pression atmosphérique, un mélange de 2,5 g du composé obtenu à l'étape précédente et 0,25 g de palladium sur charbon à 10 % dans 30 ml de MeOH. On filtre le catalyseur et concentre sous vide le filtrat. On obtient 1,7 g du produit attendu.

### 2. Préparations des composés de formule (II)

### Préparation 2.1

### 2-Chloro-1-[4-hydroxy-4-[3-(trifluorométhyl)phényl]-1-pipéridinyl]-1-éthanone.

(IIa) : R₁ = 3-CF₃ ; R₂ = H ; R₃ = -OH ; Hal = Cl.

A un mélange de 5 g du compose obtenu à la Préparation 1.1 et 10 ml de DIPEA dans 40 ml de DCM, on ajoute, goutte à goutte et à TA, 1,63 ml de chlorure de 2-chloroacétyle et laisse 30 minutes sous agitation. On lave le mélange réactionnel à l'eau, sèche la phase organique sur Na₂SO₄ et évapore le solvant sous vide. On chromatographie le résidu sur gel de silice en éluant par le mélange DCM/MeOH (97/3 ; v/v). On obtient 5,5 g du produit attendu que l'on utilise tel quel.

### Préparation 2.2

### [1-(2-Chloroacétyl)-4-[3-(trifluorométhyl)phényl]-4-pipéridinyl]méthylcarbamate de tert-butyle.

(IIa) : R₁ = 3-CF₃ ; R₂ = H ; R₃ = -CH₂NHCOOC(CH₃)₃, ; Hal = Cl.

On refroidit au bain de glace un mélange de 4,95 g du composé obtenu à la Préparation 1.3 et 6,8 ml de triéthylamine dans 50 ml de DCM, ajoute, goutte à goutte, 1,65 ml de chlorure de 2-chloroacétyle et laisse sous agitation en laissant remonter la température à TA. On concentre sous vide, reprend le résidu par une solution saturée de K₂CO₃, extrait à l'AcOEt, lave la phase organique par une solution saturée de K₂CO₃, par une solution tampon pH = 2, par une solution saturée de NaCl, sèche sur Na₂SO₄ et évapore sous vide. On chromatographie le résidu sur gel de silice en éluant par le mélange DCM/AcOEt (80/20 ; v/v). On obtient 1,8 g du produit attendu que l'on utilise tel quel.

### Préparation 2.3

### [[1-(2-chloroacétyl)-4-[3-(trifluorométhyl)phényl]-4-pipéridinyl]méthyl]méthyl-carbamate de tert-butyle.

(IIa) : R₁ = 3-CF₃ ; R₂ = H ; R₃ = -CH₂N(CH₃)COOC(CH₃)₃ ; Hal = Cl.

On refroidit à -40°C une solution de 14 g du composé obtenu à la Préparation 1.4 et 5,5 ml de triéthylamine dans 300 ml de DCM, ajoute lentement 3,1 ml de chlorure de 2-chloroacétyle et laisse sous agitation en laissant remonter la température à TA. On concentre sous vide, reprend le résidu à l'eau, extrait à l'AcOEt, lave la phase organique au tampon pH = 2, à l'eau, sèche sur Na₂SO₄ et évapore le solvant sous vide. On obtient 15,33 g du produit attendu.

### Préparation 2.4

### [[1-(2-chloroacétyl)-4-[3-(trifluorométhyl)phényl]pipéridin-4-yl]méthyl] diméthylamine.

(IIa) : R₁ = 3-CF₃ ; R₂ = H ; R₃ = -CH₂N(CH₃)₂ ; Hal = Cl.

On refroidit au bain de glace un mélange de 0,94 g du composé obtenu à la Préparation 1.5 sous forme de base libre dans 20 ml d'éther, ajoute 0,66 g de triéthylamine puis, goutte à goutte, et en 15 minutes, une solution de 0,27 ml de chlorure de 2-chloroacétyle dans 10 ml d'éther. On lave le mélange réactionnel à l'eau, sèche la phase organique sur Na₂SO₄ et utilise immédiatement la solution du produit attendu dans l'éther dans le variante de l'Exemple 5.

### Préparation 2.5

### 4-(Azétidin-1-ylcarbonyl)-1-(2-chloroacétyl)-4-[3-(trifluorométhyl)phényl] pipéridine.

(IIa) : R₁ = 3-CF₃ ; R₂ = H ; 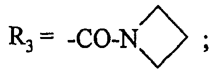 Hal = Cl.

A un mélange de 0,75 g du composé obtenu la Préparation 1.7 et 0,85 ml de DIPEA dans 10 ml de DCM, on ajoute à TA et en goutte à goutte 0,21 ml de chlorure de 2-chloroacétyle et laisse 30 minutes sous agitation à TA. On lave le mélange réactionnel par une solution tampon pH = 2, sèche la phase organique sur Na₂SO₄ et évapore le solvant sous vide. On obtient 0,9 g du produit attendu que l'on utilise tel quel.

### Préparation 2.6

### 1-(2-Chloroacétyl)-4-[3-(trifluorométhyl)phényl]-4-pipéridinecarboxamide.

(IIa) : R₁ = 3-CF₃ ; R₂ = H ; R₃ = -CONH₂ ; Hal = Cl.

A un mélange de 0,7 g du composé obtenu à la Préparation 1.8 et 0,37 ml de triéthylamine dans 10 ml de DCM et 10 ml de dioxane, on ajoute, goutte à goutte et à TA, 0,21 ml de chlorure de 2-chloroacétyle et laisse 2 heures sous agitation à TA. On concentre sous vide, reprend le résidu à l'eau, essore le précipité formé et le sèche. On obtient 0,82 g du produit attendu, F = 195-198°C.

### Préparation 2.7

### 1-[4-Hydroxy-4-[3-(trifluorométhyl)phényl]-1-pipéridinyl]-2-propèn-1-one.

(IIb) : R₁ = 3-CF₃ ; R₂ = H ; R₃ = -OH.

On refroidit au bain de glace un mélange de 5 g du composé obtenu à la Préparation 1.1 et 8 ml de triéthylamine dans 50 ml de DCM, ajoute, en goutte à goutte, 2,07 ml de chlorure de 3-bromopropionyle et laisse 2 heures sous agitation en laissant remonter la température à TA. On lave le mélange réactionnel par une solution saturée de K₂CO₃, à l'eau, sèche la phase organique sur Na₂SO₄ et évapore le solvant sous vide. On chromatographie le résidu sur gel de silice en éluant par le mélange DCM/MeOH de (98,5/1,5 ; v/v) à (97/3 ; v/v). On obtient 4,6 g du produit attendu que l'on utilise tel quel.

### EXEMPLE 1 : Composé N° 1

### Chlorhydrate de 4-[3-(trifluorométhyl)phényl]-1-[[4-[3-(trifluorométhyl)phényl] pipérazin-1-yl]acétyl]pipéridin-4-ol.

(I), HCl : R₁ = 3-CF₃ ; R₂ = H ; R₃ = -OH ; 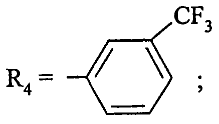 n = 1.

On laisse 4 heures sous agitation à TA un mélange de 0,8 g du composé obtenu à la Préparation 2.1, 0,6 g de 1-[3-(trifluorométhyl)phényl]pipérazine, 0,475 g d'iodure de potassium et 0,72 g de K₂CO₃ dans 40 ml d'acétonitrile. On ajoute une solution saturée de K₂CO₃ au mélange réactionnel, extrait à l'AcOEt, lave la phase organique à l'eau, par une solution saturée de NaCl, sèche sur Na₂SO₄ et évapore le solvant sous vide. On chromatographie le résidu sur gel de silice en éluant par le mélange DCM/MeOH (96/4 ; v/v). On reprend le produit obtenu dans une solution d'éther chlorhydrique 2N et, après trituration, essore le précipité formé. On obtient 1,13 g du produit attendu, F = 130°C (déc).

### EXEMPLE 2 : Composé N° 5

### Chlorhydrate de [[1-[(4-phénylpipérazin-1-yl)acétyl]-4-[3-(trifluorométhyl) phényl]pipéridin-4-yl]méthyl]amine.

(I), HCl : R₁ = 3-CF₃ ; R₂ = H ; R₃ = -CH₂NH₂ ; 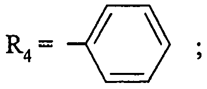 n = 1.

### A) [[1-[(4-phénylpipérazin-1-yl)acétyl]-4-[3-(trifluorométhyl)phényl]pipéridin-4-yl]méthyl]carbamate de tert-butyle.

On laisse une nuit sous agitation à TA un mélange de 4,2 g du composé obtenu à la Préparation 2.2, 1,57 g de 1-phénylpipérazine, 1,6 g d'iodure de potassium et 2,67 g de K₂CO₃ dans 40 ml d'acétonitrile. On concentre le mélange réactionnel sous vide, reprend le résidu à l'eau, extrait à l'éther, sèche la phase organique sur Na₂SO₄ et évapore le solvant sous vide. On obtient 7 g du produit attendu que l'on utilise tel quel.

### B) Chlorhydrate de [[1-[(4-phénylpipérazin-1-yl)acétyl]-4-[3-(trifluorométhyl) phényl]pipéridin-4-yl]méthyl]amine.

On laisse 30 minutes sous agitation à TA un mélange de 7 g du composé obtenu à l'étape précédente, 100 ml d'éther chlorhydrique 2N et 50 ml de MeOH. On rajoute 20 ml d'une solution d'HCl à 30 % et laisse 1 heure sous agitation à TA. On concentre le mélange réactionnel sous vide, reprend le résidu par une solution de NaOH à 10 %, extrait au DCM, sèche la phase organique sur Na₂SO₄ et évapore le solvant sous vide. On chromatographie le résidu sur gel de silice H en éluant par le mélange DCM/MeOH/H₂O de (100/5/0,5 ; v/v/v) à (100/10/1 ; v/v/v). On obtient 3,5 g du produit attendu sous forme de base libre. On reprend 0,4 g du produit obtenu dans une solution d'éther chlorhydrique 2N, laisse sous agitation et essore le précipité formé. On obtient 0,33 g du produit attendu, F = 185-200°C.
Spectre de masse : MH⁺ = 461,2.

### EXEMPLE 3 : Composé N° 6

### (2-Furylméthyl)[[1-[(4-phénylpipérazin-1-yl)acétyl]-4-[3-(trifluorométhyl) phényl]pipéridin-4-yl]méthyl]amine.

(1): R₁ = 3-CF_{3 ;} R₂ = H _{;} 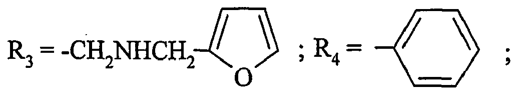 n = 1.

A un mélange de 0,67 g du composé N° 5 sous forme de base libre, 0,14 g de 2-furaldéhyde et 3 gouttes d'acide acétique dans 30 ml de THF, on ajoute, par portions, 0,62 g de triacétoxyborohydrure de sodium et laisse une nuit sous agitation à TA. On concentre le mélange réactionnel sous vide, reprend le résidu dans 10 ml de MeOH et chauffe à 80°C pendant 30 minutes. On concentre sous vide, reprend le résidu par une solution de NaOH à 10 %, extrait au DCM, sèche la phase organique sur Na₂SO₄ et évapore le solvant sous vide. On chromatographie le résidu sur gel de silice H en éluant par le mélange DCM/MeOH de (100/1 ; v/v) à (100/5 ; v/v). On obtient 0,24 g du produit attendu après cristallisation dans l'éther iso, F = 119°C.
Spectre de masse : MH+ = 541,2.

### EXEMPLE 4 : Composé N° 11

### Dichlorhydrate de N-méthyl-1-[1-[(4-phénylpipérazin-1-yl)acétyl]-4-[3-(trifluorométhyl)phényl]pipéridin-4-yl]méthanamine, 3 H₂O.

(I), 2 HCl : R₁ = 3-CF_{3 ;} R₂ = H _{;} R₃ = -CH₂NHCH_{3 ;} 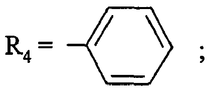 n = 1.

### A) [[1-[(4-Phénylpipérazin-1-yl)acétyl]-4-[3-(trifluorométhyl)phényl]pipéridin-4-yl]méthyl]carbamate de tert-butyle méthyle.

On laisse 3 jours sous agitation à TA un mélange de 1,98 g du composé obtenu à la Préparation 2.3, 0,74 g de 1-phénylpipérazine, 0,5 g d'iodure de potassium et 2,0 g de K₂CO₃ dans 20 ml d'acétonitrile. On concentre le mélange réactionnel sous vide, reprend le résidu par une solution saturée de K₂CO₃, extrait à l'AcOEt, lave la phase organique à l'eau, sèche sur Na₂SO₄ et évapore le solvant sous vide. On chromatographie le résidu sur gel de silice en éluant par le mélange DCM/MeOH (97/3 ; v/v). On obtient 1,6 g du produit attendu que l'on utilise tel quel.

### B) Dichlorhydrate de N-méthyl-1-[1-[(4-phénylpipérazin-1-yl)acétyl]-4-[3-(trifluorométhyl)phényl]pipéridin-4-yl]méthanamine, 3 H₂O.

A une solution de 1,6 g du composé obtenu à l'étape précédente dans 10 ml de méthanol, on ajoute 1 goutte d'eau, puis, en 1heure, 50 ml d'une solution d'éther chlorhydrique 2N et laisse 24 heures sous agitation à TA. On essore le précipité formé et le lave à l'éther. On obtient 1,2 g du produit attendu, F = 190-200°C.

### EXEMPLE 5 : Composé N° 12

### N,N-Diméthyl-1-[1-[(4-phériylpipérazin-1-yl)acétyl]-4-[3-(trifluorométhyl) phényl]pipéridin-4-yl]méthanamine.

(I) : R₁ = 3-CF_{3 ;} R₂ = H _{;} R₃ = -CH₂N(CH₃)_{2 ;} 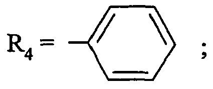 n = 1.

A une suspension de 0,5 g du composé N° 11 dans 20 ml de THF, on ajoute, à TA, 0,5 ml d'acide acétique puis 0,14 ml d'une solution de formaldéhyde à 37 % dans l'eau et 0,36 g de triacétoxyborohydrure de sodium et laisse 3 heures sous agitation à TA. On concentre le mélange réactionnel sous vide, reprend le résidu par une solution saturée de K₂CO₃, extrait à l'AcOEt, lave la phase organique à l'eau, sèche sur Na₂SO₄ et évapore le solvant sous vide. On chromatographie le résidu sur gel de silice en éluant par le mélange DCM/MeOH (95/5 ; v/v). On obtient 0,25 g du produit attendu après cristallisation dans DCM/éther iso, F = 123-124°C.

On peut également préparer le composé N° 12 en suivant le mode opératoire ci-après.

A une solution de 0,53 g de 1-phénylpipérazine, 0,12 g d'iodure de potassium et 1,2 g de K₂CO₃ dans 5 ml d'acétonitrile, on ajoute la solution du composé de la Préparation 2.4 dans l'éther et laisse une nuit sous agitation à TA. On concentre le mélange réactionnel sous vide, reprend le résidu par une solution saturée de K₂CO₃, extrait à l'AcOEt, lave la phase organique à l'eau, par une solution saturée de NaCl, sèche sur Na₂SO₄ et évapore le solvant sous vide. On chromatographie le résidu sur gel de silice en éluant par le mélange DCM/MeOH (96/4 ; v/v). On obtient 0,932 g du produit attendu après cristallisation dans le mélange DCM/éther iso, F = 122°C.

### EXEMPLE 6 : Composé N° 13

### Dichlorhydrate de N-méthyl-N-[[1-[(4-phénylpipérazin-1-yl)acétyl]-4-[3-(trifluorométhyl)phényl]pipéridin-4-yl]méthyl]éthanamine, 3 H₂O.

(I), 2 HCl : R₁ = 3-CF_{3 ;} R₂ = H _{;} R₃ = -CH₂N(CH₃)CH₂CH_{3 ;} 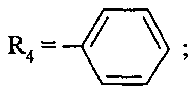 n = 1.

A une suspension de 0,5 g du composé N° 11 et 0,5 ml d'acide acétique dans 10 ml de THF, on ajoute, à TA, 0,1 ml d'acétaldéhyde puis, en une fois, 0,36 g de triacétoxyborohydrure de sodium et laisse 24 heures sous agitation à TA. On concentre le mélange réactionnel sous vide, reprend le résidu dans 20 ml de MeOH et chauffe à 65°C pendant 3 heures. On concentre sous vide, reprend le résidu par une solution saturée de K₂CO₃, extrait à lacet, lave la phase organique à l'eau, sèche sur Na₂SO₄ et évapore le solvant sous vide. On chromatographie le résidu sur gel de silice en éluant par le mélange DCM/MeOH (96/4 ; v/v). On dissout le produit obtenu dans 10 ml d'éther et 1 goutte de MeOH, ajoute une solution d'éther chlorhydrique 2N et essore le précipité formé. On obtient 0,18 g du produit attendu.
Spectre de masse : MH⁺ = 503,4.

### EXEMPLE 7 : Composé N° 20

### Trichlorhydrate de [[1-[[4-(3,4-diméthoxyphényl)pipérazin-1-yl]acétyl]-4-[3-(trifluorométhyl)phényl]pipéridin-4-yl]méthyl]diméthylamine, 2 H₂O.

(I), 3 HCl : R₁ = 3-CF_{3 ;} R₂ = H _{;} R₃ = -CH₂N(CH₃)_{2 ;} 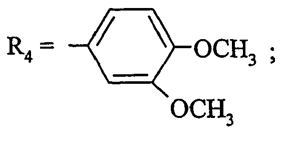 n = 1.

A une solution de 0,65 g du composé N° 19 et 0,5 ml d'acide acétique dans 10 ml de THF, on ajoute 0,5 ml d'une solution de formaldéhyde à 37 % dans l'eau, puis, en une fois, 1,25 g de triacétoxyborohydrure de sodium et laisse une nuit sous agitation à TA. On rajoute ensuite 0,25 ml d'acide acétique, 0,25 ml d'une solution de formaldéhyde à 37 % dans l'eau et 0,55 g de triacétoxyborohydrure de sodium et laisse 24 heures sous agitation à TA. On concentre sous vide, reprend le résidu dans 40 ml de MeOH et chauffe à 65°C pendant 5 heures. On concentre sous vide, reprend le résidu par une solution saturée de K₂CO₃, extrait à l'AcOEt, lave la phase organique à l'eau, sèche sur Na₂SO₄ et évapore le solvant sous vide. On chromatographie le résidu sur alumine en éluant par le mélange DCM/MeOH (98/2 ; v/v). On reprend le produit obtenu dans une solution d'éther chlorhydrique 2N, laisse sous agitation et essore le précipité formé. On obtient 0,33 g du produit attendu, F = 190-210°C.

### EXEMPLE 8 : Composé N° 21

### Dichlorhydrate de N-éthyl-N-[[1-[[4-(3-méthoxyphényl)pipérazin-1-yl]acétyl]-4-[3-(trifluorométhyl)phényl]pipéridin-4-yl]méthyl]éthanamine.

(I), 2 HCl : R₁ = 3-CF_{3 ;} R₂ = H _{;} R₃ = -CH₂N(CH₂CH₃)_{2 ;} 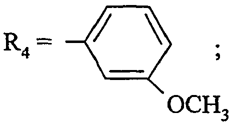 n = 1.

A une suspension de 0,5 g du composé N° 15 et 0,5 ml d'acide acétique dans 8 ml de THF, on ajoute, à TA, 0,15 ml d'acétaldéhyde puis 0,56 g de triacétoxyborohydrure de sodium et laisse 24 heures sous agitation à TA. On concentre le mélange réactionnel sous vide, reprend le résidu dans 30 ml de MeOH et chauffe à 65°C pendant 3 heures. On concentre sous vide, reprend le résidu par une solution saturée de K₂CO₃, extrait à l'AcOEt, lave la phase organique à l'eau, sèche sur Na₂SO₄ et évapore le solvant sous vide. On chromatographie le résidu sur gel de silice en éluant par le mélange DCM/MeOH (97/3 ; v/v). On reprend le produit obtenu dans une solution d'éther chlorhydrique 2N, laisse sous agitation et essore le précipité formé. On obtient 0,14 g du produit attendu.
Spectre de masse : MH⁺ = 547,2.

### EXEMPLE 9 : Composé N° 22

### 1-[2-[4-(Azétidin-1-ylcarbonyl)-4-[3-(trifluorométhyl)phényl]pipéridin-1-yl]-2-oxoéthyl]-4-phénylpipérazine.

(I) : R₁ = 3-CF_{3 ;} R₂ = H _{;} 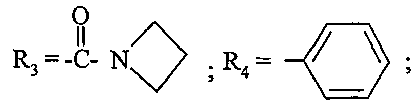 n = 1.

On laisse 2 heures sous agitation à TA un mélange de 0,42 g du composé obtenu à la Préparation 2.5, 0,2 g de 1-phénylpipérazine, 0,2 g d'iodure de potassium et 0,3 g de K₂CO₃ dans 10 ml d'acétonitrile. On ajoute une solution saturée de K₂CO₃ au mélange réactionnel, extrait au DCM, lave la phase organique à l'eau, sèche sur Na₂SO₄ et évapore le solvant sous vide. On chromatographie le résidu sur gel de silice en éluant par le mélange DCM/MeOH (97/3 ; v/v). On obtient 0,25 g du produit attendu après cristallisation dans le mélange DCM/éther iso, F = 147-149°C.
Spectre de masse : MH⁺ = 515,6.

Le tableau qui suit illustre les structures chimiques et les propriétés physiques de quelques exemples de composés selon l'invention. Dans ce tableau :
- dans la colonne "sel", "-" représente un composé sous forme de base libre, alors que "HCl" représente un composé sous forme de chlorhydrate et "C₂H₂O₄" représente un composé sous forme d'oxalate.

**TABLEAU I**

| | | | | | | | |
|---|---|---|---|---|---|---|---|
| 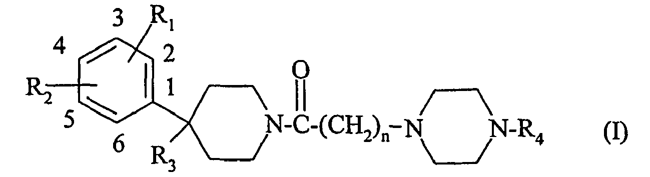 | | | | | | | |

| Composés N° | R₁ | R₂ | R₃ | n | R₄ | Sel; hydrate | F°C ; Solvant de cristallisation ; MH⁺ |
|---|---|---|---|---|---|---|---|
| 1 | 3-CF₃ | H | -OH | 1 | 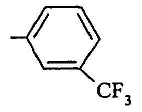 | HCl ; 1H₂O | 130; éther ; - |
| 2 (a) | 3-CF₃ | H | -OH | 1 | 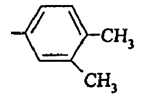 | C₂H₂O₄ | 193-196 ; éther ; - |
| 3 (a) | 3-CF₃ | H | -OH | 1 | 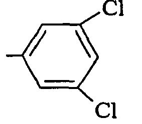 | - | - - 516 |
| 4 (a) | 3-CF₃ | H | -OH | 1 | 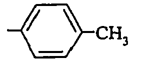 | - | - - 462 |
| 5 | 3-CF₃ | H | -CH₂NH₂ | 1 | 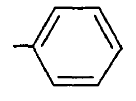 | 3HCl ; 1,5H₂O | 185-200; éther ; 461,2 |
| 6 | 3-CF₃ | H | 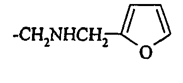 | 1 | 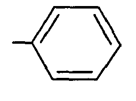 | - | 119 ; éther iso; 541,2 |
| 7 (b) | 3-CF₃ | H | 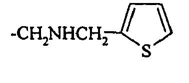 | 1 | 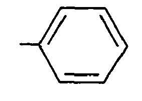 | - | 141 ; éther ; 557,2 |
| 8 (b) | 3-CF₃ | H | 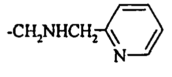 | 1 | 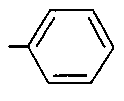 | C₂H₂O₄ 0,5H₂O | 152-160; éther; 552,4 |
| 9 (b) | 3-CF₃ | H | 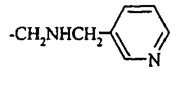 | 1 | 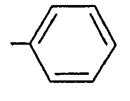 | 2C₂H₂O₄ 0,5H₂O | 85-100 ; éther; 552,7 |
| 10 (b) | 3-CF₃ | H | 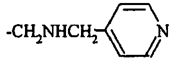 | 1 | 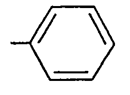 | C₂H₂O₄ | 108-118 ; éther ; 552,4 |
| 11 | 3-CF₃ | H | -CH₂NHCH₃ | 1 | 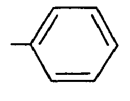 | 2HCl ; 3H₂O | 190-200 ; MeOH/éther ; - |
| 12 | 3-CF₃ | H | -CH₂N(CH₃)₂ | 1 | 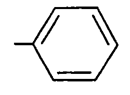 | - | 122; DCM/éther iso ; 489,4 |
| 13 | 3-CF₃ | H | -CH₂N(CH₃)- -CH₂CH₃ | 1 | 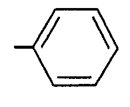 | 2HCl ; 3H₂O | - éther ; 503,4 |
| 14 (c) | 3-CF₃ | H | -CH₂NH₂ | 1 | 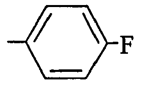 | 3HCl ; 2H₂O | - éther; 479,2 |
| 15 (c) | 3-CF₃ | H | -CH₂NH₂ | 1 | 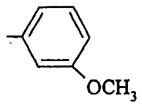 | 2HCl ; 3H₂O | - MeOH/éther ; 491,4 |
| 16 (c) | 3-CF₃ | H | -CH₂NH₂ | 1 | 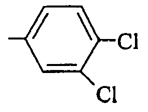 | - | 109-110 ; DCM/éther iso ; - |
| 17 (d) | 3-CF₃ | H | -CH₂NHCH₃ | 1 | 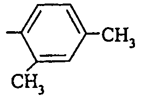 | 2HCl ; 3H₂O | 204-211 ; éther ; 503,7 |
| 18 (e) | 3-CF₃ | H | -CH₂N(CH₃)₂ | 1 | 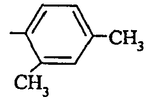 | 2HCl, 5H₂O | - éther ; 517,4 |
| 19 (c) | 3-CF₃ | H | -CH₂NH₂ | 1 | 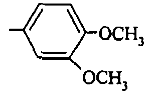 | 3HCl ; 2H₂O | 208 ; MeOH/éther ; 521,5 |
| 20 | 3-CF₃ | H | -CH₂N(CH₃)₂ | 1 | 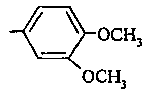 | 3HCl ; 2H₂O | 190-210 ; éther ; - |
| 21 | 3-CF₃ | H | -CH₂N(CH₂CH₃)₂ | 1 | 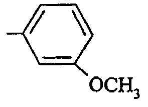 | 2HCl ; 3,5H₂O | - éther 547,2 |
| 22 | 3-CF₃ | H | 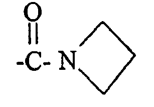 | 1 | 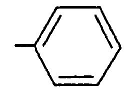 | - | 147-149 ; DCM/éther iso ; 515,6 |
| 23 | 3-CF₃ | H | -CH₂NHCH₃ | 1 | 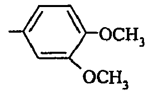 | 2HCl ; 3H₂O | - éther; 535,2 |
| 24 (a) | 3-CF₃ | H | -OH | 1 | 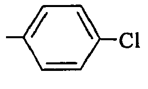 | - | - - 482,1 |
| 25 (a) | 3-CF₃ | H | -OH | 1 | 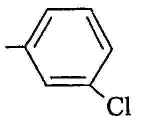 | - | - - 482,1 |
| 26 (a) | 3-CF₃ | H | -OH | 1 | 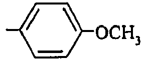 | - | - - 478,2 |
| 27 (a) | 3-CF₃ | H | -OH | 1 | 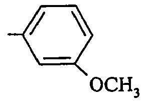 | - | - - 478,2 |
| 28 (f) | 3-CF₃ | H | -CONH₂ | 1 | 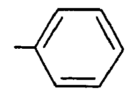 | - | - - 474,8 |
| 29 (f) | 3-CF₃ | H | -CONH₂ | 1 | 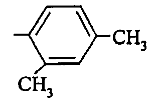 | - | - - 502,8 |
| 30 (f) | 3-CF₃ | H | -CONH₂ | 1 | 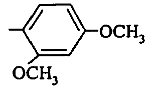 | - | - - 534,8 |
| 31 (f) | 3-CF₃ | H | -CONH₂ | 1 | 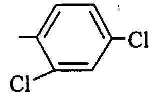 | - | - - 542,7 |
| 32 (g) | 3-CF₃ | H | -OH | 2 | 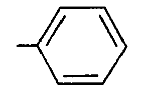 | - | - - 461,8 |
| 33 (g) | 3-CF₃ | H | -OH | 2 | 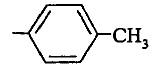 | - | - - 475,8 |
| 34 (g) | 3-CF₃ | H | -OH | 2 | 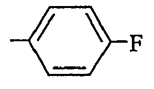 | - | - - 479,7 |
| 35 (g) | 3-CF₃ | H | -OH | 2 | 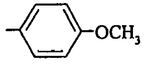 | - | - - 491,8 |
| 36 (h) | 3-CF₃ | H | 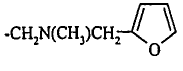 | 1 | 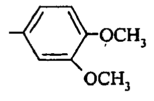 | 3HCl, 2H₂O | - - 615 |
| 37 (b) | 3-CF₃ | H | 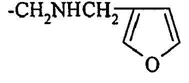 | 1 | 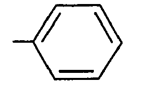 | - | 137 ; éther iso ; 541,2 |
| 38 (c) | 3-CF₃ | H | -CH₂NH₂ | 1 | 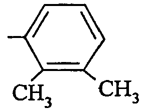 | - | 114-116 ; DCM/éther iso 489,6 |

| | | | | | | | |
|---|---|---|---|---|---|---|---|
| (a) Composé préparé selon le mode opératoire décrit à l'Exemple 1 à partir du composé de la Préparation 2.1 et du composé de formule (III) correspondant. (b) Composé préparé selon le mode opératoire décrit à l'Exemple 3 à partir du composé N° 5 et du composé de formule (IV) correspondant. (c) Composé préparé selon les modes opératoires décrits aux étapes A et B de l'Exemple 2 à partir du composé obtenu à la Préparation 2.2 et du composé de formule (III) correspondant. (d) Composé préparé selon les modes opératoires décrits aux étapes A et B de l'Exemple 4 à partir du composé obtenu à la Préparation 2.3 et du composé de formule (III) correspondant. (e) Composé préparé selon le mode opératoire décrit à l'Exemple 5 à partir du composé de formule (I) dans laquelle R₃ = CH₂NHCH₃ correspondant. (f) Composé préparé selon le mode opératoire décrit à l'Exemple 1 à partir du composé obtenu à la Préparation 2.6 et du composé de formule (III) correspondant. (g) Composé préparé selon le mode opératoire décritsà l'Exemple 1 à partir du composé obtenu à la Préparation 2.7 et du composé de formule (III) correspondant. (h) Composé préparé selon le mode opératoire décrit à l'Exemple 3 à partir du composé N° 23 et du composé de formule (IV) correspondant. | | | | | | | |

Les composés selon l'invention ont fait l'objet d'études biochimiques.

### Culture cellulaire :

La souche SH-SY-5Y (neuroblastome humain) est cultivée classiquement dans un milieu de culture DMEM (de l'anglais Dulbecco's Modified Eagle's Medium)(Gibco BRL, France) contenant du SVF (5%) (serum de veau foetal) (Boehringer Mannheim, Germany), du pyruvate de sodium (1 mM), de l'anti-PPLO( 5 ml) (agent anti mycoplasme : Tylocine^{®} préparée dans une solution saline normale, 6000 µg/ml), de la gentamycine (0.1mg/ml) et de la glutamine (4 mM) dans des flacons de culture recouvert de collagène (Becton Dickinson, France).

La souche mère SK-N-BE (neuroblastome humain) et le clone Bep 75 exprimant le récepteur p75^{NTR} humain (SK-N-BE Bep 75) sont cultivés classiquement dans un milieu de culture DMEM contenant du SVF (5%), du pyruvate de sodium (1 mM), de l'anti-PPLO( 5 ml), de la gentamycine (0.1mg/ml) et de la glutamine (4 mM).

### Etude de la liaison du ¹²⁵I NGF au récepteur P75^{NTR}

L'étude de la liaison du ¹²⁵I NGF (le facteur de croissance neuronale radio marqué à l'iode-125) est réalisée sur une suspension cellulaire des deux souches SH-SY-5Y et SK-N-BE Bep 75 en accord avec la méthode décrite par Weskamp (Neuron, 1991, 6, 649-663). La liaison non spécifique est déterminée par la mesure de la liaison totale après une heure de pré-incubation avec les cellules à 37°C en présence de NGF non-radio marqué (1 µM). La liaison spécifique est calculée par différence entre la mesure de la liaison totale et la mesure de liaison non-spécifique. Les expériences de compétition sont réalisées en utilisant une concentration en ¹²⁵I NGF de 0.3 nM. Les concentrations inhibitrices de 50 % (CI₅₀) de la fixation de ¹²⁵I NGF au récepteur p75^{NTR} des composés selon l'invention sont faibles et varient de 10⁻⁶ à 10⁻¹¹M.

### Mesure de l'apoptose :

Les cellules (souches de neuroblastomes humains SH-SY-5Y et SK-N-BE Bep 75) sont installées dans des boîtes de Pétri de 35 mm de diamètre (Biocoat collagen I, (10⁵ cellules/puits) dans un milieu de culture DMEM contenant 5 % de SVF durant 24H. Le milieu de culture est ensuite éliminé, les cellules sont rincées avec du PBS (de l'anglais Dulbecco's Phosphate buffered saline) et soit du milieu frais contenant 5% de SVF soit du milieu contenant du NGF à la concentration de 10 ng/ml est ajouté en présence ou non des composés selon l'invention. Les taux d'apoptose sont mesurés 48 heures après les traitements dans le cas de la souche SH-SY-5Y et 24 heures après dans le cas de la souche SK-N-BE Bep 75 par quantification des histones cytoplasmiques associés aux fragments d'ADN (cell death detection ELISA, Boehringer Mannheim, Germany). Les taux d'apoptose sont exprimés en quantité d'oligonucléosomes/105 cellules ± DS. Chaque valeur correspond à la moyenne de 9 points expérimentaux répartis dans 3 expériences indépendantes. Les composés de formule (I) présentent une activité inhibitrice de l'apoptose induite par le NGF avec des CI₅₀ qui varient de 10⁻⁶ à 10⁻¹¹M.

Ainsi la fixation des composés selon l'invention au récepteur p75^{NTR} se traduit d'une part au niveau biochimique par l'inhibition de la dimérisation du récepteur induit par les neurotrophines et d'autre part au niveau cellulaire par l'inhibition de l'effet proapoptotique médié par le récepteur p75^{NTR}.

Les composés selon l'invention peuvent donc être utilisés pour la préparation de médicaments, en particulier de médicaments destinés à prévenir ou à traiter toute pathologie où le récepteur p75^{NTR} est impliqué.

Ainsi, selon un autre de ses aspects, l'invention a pour objet des médicaments qui comprennent un composé de formule (I), ou un sel d'addition de ce dernier à un acide pharmaceutiquement acceptable, ou encore un solvat ou un hydrate du composé de formule (I).

Ainsi, les composés selon l'invention peuvent être utilisés, chez l'homme ou chez l'animal, dans le traitement ou la prévention de différentes affections p75^{NTR} dépendantes telles que les maladies neurodégénératives centrales et périphériques comme la démence sénile, l'épilepsie, la maladie d'Alzheimer, la maladie de Parkinson, la chorée d'Huntington, le syndrôme de Down, les maladies à prion, l'amnésie, la schizophrénie ; la sclérose latérale amyotrophique, la sclérose en plaques ; les affections cardiovasculaires comme les dommages cardiaques post-ischémiques, les cardiomyopathies, l'infarctus du myocarde, l'insuffisance cardiaque, l'ischémie cardiaque, l'infarctus cérébral ; les neuropathies périphériques (d'origine diabétique, traumatisme ou iatrogène) ; les dommages au nerf optique et à la rétine; les traumatismes de la moëlle épinière et les traumatismes crâniens ; l'athérosclérose; les sténoses ; la cicatrisation ; l'alopécie.

Les composés selon l'invention peuvent également être utilisés dans le traitement des cancers comme celui du poumon, de la thyroïde, du pancréas, de la prostate, de l'intestin grêle et du colon, du sein, dans le traitement des tumeurs, des métastases et des leucémies.

Les composés selon l'invention peuvent aussi être utilisés dans le traitement des douleurs chroniques neuropathiques et inflammatoires et dans le traitement des maladies auto-immunes comme la polyarthrite rhumatoïde.

Les composés selon l'invention peuvent également être utilisés dans le traitement des fractures osseuses, dans le traitement ou la prévention des maladies osseuses comme l'ostéoporose.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | | |
|---|---|---|
| Composé selon l'invention | : | 50,0 mg |
| Mannitol | : | 223,75 mg |
| Croscarmellose sodique | : | 6,0 mg |
| Amidon de maïs | : | 15,0 mg |
| Hydroxypropyl-méthylcellulose | : | 2,25 mg |
| Stéarate de magnésium | : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) : 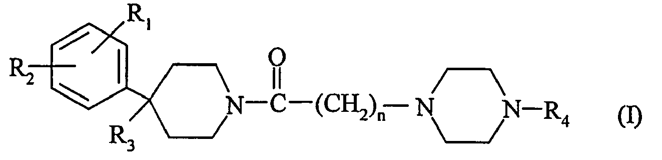 dans laquelle :
- n est 1 ou 2 ;
- R₁ représente un atome d'halogène ; un radical trifluorométhyle ; un (C₁-C₄)alkyle ; un (C₁-C₄)alcoxy ; un radical trifluorométhoxy ;
- R₂ représente un atome d'hydrogène ou un atome d'halogène ;
- R₃ représente un atome d'hydrogène ; un groupe -OR₅ ; un groupe -CH₂OR_{S} ; un groupe -NR₆R₇ ; un groupe NR₈COR₉ ; un groupe -CH₂NR₁₀R¹¹; un (C₁-C₄)alcoxycarbonyle ; un groupe -CONR₁₂R_{13 ;} un radical -CN ;
- R₄ représente un phényle non substitué, mono-, di- ou trisubstitué par un substituant choisi indépendamment parmi un atome d'halogène, un (C₁-C₄)alkyle, un (C₁-C₄)alcoxy, un radical trifluorométhyle ;
- R₅ représente un atome d'hydrogène ou un (C₁-C₄)alkyle;
- R₆ et R₇ représentent chacun indépendamment un atome d'hydrogène ou un (C₁-C₄)alkyle ;
- R₈ représente un atome d'hydrogène ou un (C₁-C₄)alkyle ;
- R₉ représente un (C₁-C₄)alkyle ;
- R₁₀ et R₁₁ représentent chacun indépendamment un atome d'hydrogène ou un (C1-C4)ₐlkyle ; R₁₁ peut de plus représenter un groupe -CH₂R₁₄ ;
- ou bien R₁₀ et R₁₁ ensemble avec l'atome d'azote auquel ils sont liés constituent un hétérocycle choisi parmi l'aziridine, l'azétidine, la pyrrolidine, la pipéridine ou la morpholine ;
- R₁₂ et R₁₃ représente chacun indépendamment un atome d'hydrogène ou un (C₁-C₄)alkyle ;
- ou bien R₁₂ et R₁₃ ensemble avec l'atome d'azote auquel ils sont liés constituent un hétérocycle choisi parmi l'azétidine, la pyrrolidine, la pipéridine, la morpholine ou la pipérazine non substituée ou substituée en position -4- par un (C₁-C₄)alkyle ;
- R₁₄ représente un groupe aromatique choisi parmi : 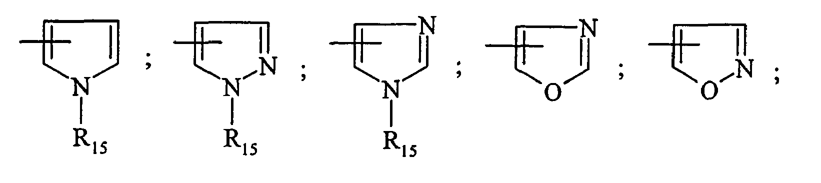 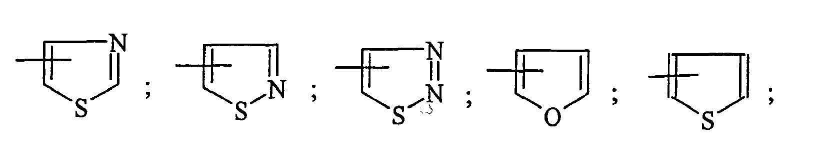 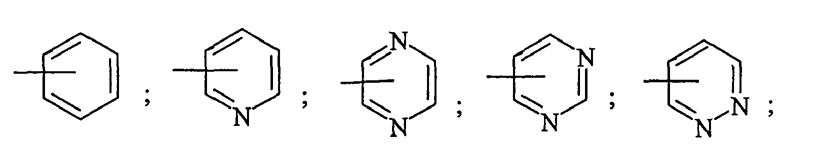 lesdits groupes aromatiques étant non substitués, mono- ou disubstitués par un substituant choisi indépendamment parmi un atome d'halogène, un (C₁-C₃)alkyle ou un (C₁-C₃)alcoxy ;
- R₁₅ représente un atome d'hydrogène ou un (C₁-C₃)alkyle ;
à l'état de base ou de sel d'addition à un acide, ainsi qu'à l'état d'hydrate ou de solvat.

2. Composé de formule (I) selon la revendication 1, **caractérisé en ce que** :
- n est 1 ou 2 ;
à l'état de base ou de sel d'addition à un acide, ainsi qu'à l'état d'hydrate ou de solvat.

3. Composé de formule (I) selon la revendication 1, **caractérisé en ce que** :
- R₁ est en position -3- du phényle et représente un radical trifluorométhyle ;
à l'état de base ou de sel d'addition à un acide, ainsi qu'à l'état d'hydrate ou de solvat.

4. Composé de formule (I) selon la revendication 1, **caractérisé en ce que** :
- R₂ représente un atome d'hydrogène ;
à l'état de base ou de sel d'addition à un acide, ainsi qu'à l'état d'hydrate ou de solvat.

5. Composé de formule (I) selon la revendication 1, **caractérisé en ce que** :
- R₃ représente un hydroxy, un aminométhyle, un (2-furylméthyl amino)méthyle, un (2-thiénylméthylamino)méthyle, un (2-pyridinylméthylamino)méthyle, un (3-pyridinylméthylamino)méthyle, un (4-pyridinylméthylamino)méthyle, un (méthylamino)méthyle, un (diméthylamino)méthyle, un (N-méthyléthylamino)méthyle, un (diéthylamino)méthyle, un azétidin-1-ylcarbonyle ; un aminocarbonyle ; un (N-méthyl-2-furylméthylamino)méthyle ; un (3-furylméthylamino)méthyle ;
à l'état de base ou de sel d'addition à un acide, ainsi qu'à l'état d'hydrate ou de solvat.

6. Composé de formule (I) selon la revendication 1, **caractérisé en ce que** :
- R₄ représente un phényle, un 4-chlorophényle, un 3-chlorophényle, un 4-fluorophényle, un 2,4-dichlorophényle, un 3,4-dichlorophényle, un 3,5-dichlorophényle, un 4-méthylphényle, un 2,4-diméthylphényle, un 3,4-diméthylphényle, un 3-méthoxyphényle, un 4-méthoxyphényle, un 2,4-diméthoxyphényle, un 3,4-diméthoxyphényle, un 3-(trifluorométhyl)phényle ; un 2,3-diméthylphényle ;
à l'état de base ou de sel d'addition à un acide, ainsi qu'à l'état d'hydrate ou de solvat.

7. Composé de formule (I) selon la revendication 1, **caractérisé en ce que** :
- n est 1 ou 2;
- R₁ est en position -3- du phényle et représente un radical trifluorométhyle ;
- R₂ représente un atome d'hydrogène ;
- R₃ représente un hydroxy, un aminométhyle, un (2-furylméthyl amino)méthyle, un (2-thiénylméthylamino)méthyle, un (2-pyridinylméthylamino)méthyle, un (3-pyridinylméthylamino)méthyle, un (4-pyridinylméthylamino)méthyle, un (méthylamino)méthyle, un (diméthylamino)méthyle, un (N-méthyléthylamino) méthyle, un (diéthylamino)méthyle, un azétidin-1-ylcarbonyle ; un aminocarbonyle ; un (N-méthyl-2-furylméthylamino)méthyle ; un (3-furylméthylamino)méthyle ;
- R₄ représente un phényle, un 4-chlorophényle, un 3-chlorophényle, un 4-fluorophényle, un 2,4-dichlorophényle, un 3,4-dichlorophényle, un 3,5-dichlorophényle, un 4-méthylphényle, un 2,4-diméthylphényle, un 3,4-diméthylphényle, un 3-méthoxyphényle, un 4-méthoxyphényle, un 2,4-diméthoxyphényle, un 3,4-diméthoxyphényle, un 3-(trifluorométhyl)phényle ; un 2,3-diméthylphényle ;
à l'état de base ou de sel d'addition à un acide, ainsi qu'à l'état d'hydrate ou de solvat.

8. Procédé de préparation des composé de formule (I) selon la revendication 1 dans laquelle n = 1, **caractérisé en ce que** :
a1) on fait réagir un composé de formule : 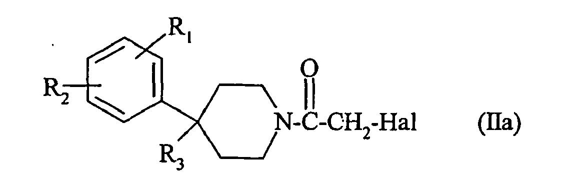 dans laquelle R₁, R₂ et R₃ sont tels que définis pour un composé de formule (I) dans la revendication 1 et Hal représente un atome d'halogène, le chlore ou le brome de préférence, étant entendu que lorsque R₃ contient une fonction hydroxyle ou amine, ces fonctions peuvent être protégées, avec un composé de formule : 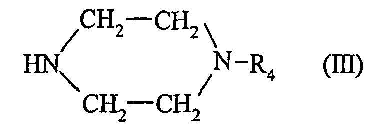 dans laquelle R₄ est tel que défini pour un composé de formule (I) dans la revendication 1 ;
b1) et, après déprotection éventuelle des fonctions hydroxyle ou amine contenues dans R₃, on obtient le composé de formule (I).

9. Procédé de préparation des composés de formule (I) selon la revendication 1 dans laquelle n = 2 , **caractérisé en ce que** :
a2) on fait réagir un composé de formule : 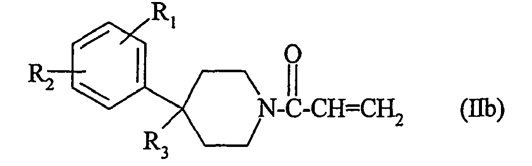 dans laquelle R₁, R₂ et R₃ sont tels que définis pour un composé de formule (I) dans la revendication 1, étant entendu que lorsque R₃ contient une fonction hydroxyle ou amine, ces fonctions peuvent être protégées, avec un composé de formule : 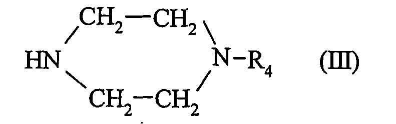 dans laquelle R₄ est tel que défini pour un composé de formule (I) dans la revendication 1 ;
b2) et, après déprotection éventuelle des fonctions hydroxyle ou amine contenues dans R₃, on obtient le composé de formule (I).

10. Procédé de préparation des composés de formule (I) selon la revendication 1 dans laquelle R₃ représente un groupe -CH₂NR₁₀R₁₁ dans lequel R₁₀ et R₁₁ représentent chacun l'hydrogène, **caractérisé en ce que** :
a3) on fait réagir un composé de formule : 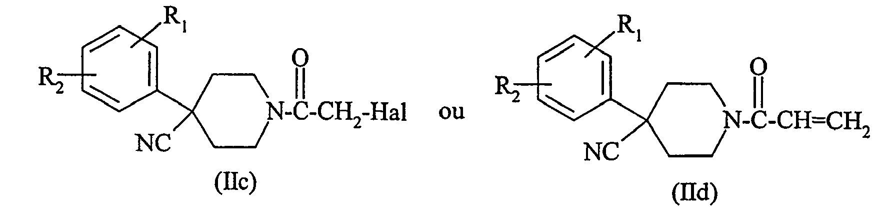 dans laquelle R₁ et R₂ sont tels que définis pour un composé de formule (I) dans la revendication 1, et Hal représente un atome d'halogène, de préférence le chlore ou le brome, avec un composé de formule : 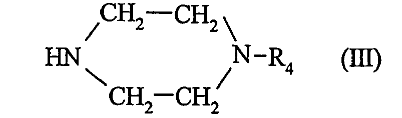 dans laquelle R₄ est tel que défini pour un composé de formule (I) dans la revendication 1, pour obtenir un composé de formule : 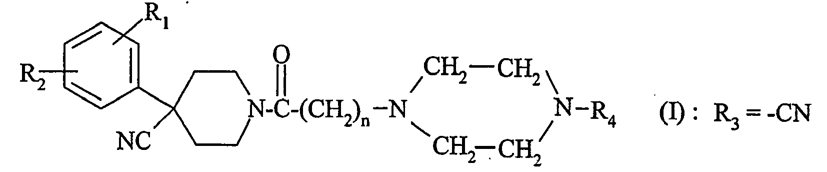
b3) on réduit le groupe cyano du composé de formule (I) pour obtenir un composé de formule (I) selon la revendication 1 dans laquelle R₃ = CH₂NH₂.

11. Procédé de préparation des composés de formule (I) selon la revendication 1 dans laquelle R₃ représente un groupe -CH₂NR₁₀R₁₁ dans lequel R₁₁ représente un groupe -CH2^{R}14 ^{:}
a4) on fait réagir un composé de formule : 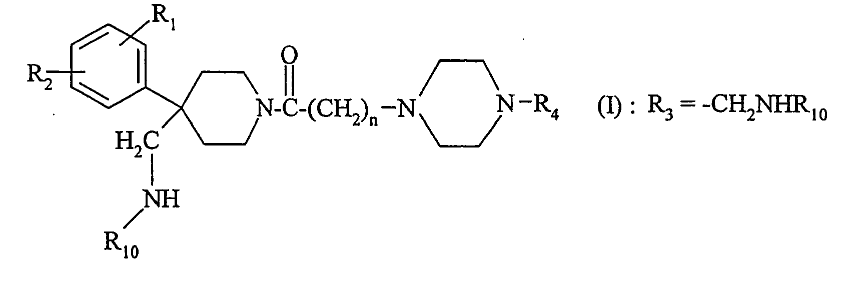 dans laquelle R₁, R₂, R₄, R₁₀ et n sont tels que définis pour un composé de formule (I) dans la revendication 1, avec un composé de formule : 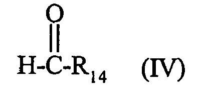 dans laquelle R₁₄ est tel que défini pour un composé de formule (I) dans la revendication 1, en présence d'un acide, dans un solvant, puis on réduit le sel d'iminium formé intermédiairement au moyen d'un agent réducteur.

12. Médicament, **caractérisé en ce qu'**il comprend un composé de formule (I) selon l'une quelconque des revendications 1 à 7, ou un sel d'addition de ce composé à un acide pharmaceutiquement acceptable, ou encore un hydrate ou un solvat du composé de formule (I).

13. Composition pharmaceutique, **caractérisé en ce qu'**elle comprend un composé de formule (I) selon l'une quelconque des revendications 1 à 7, ou un sel pharmaceutiquement acceptable, un hydrate ou un solvat de ce composé, ainsi qu'au moins un excipient pharmaceutiquement acceptable.

14. Utilisation d'un composé de formule (I) selon l'une quelconque des revendications 1 à 7 pour la préparation d'un médicament destiné à la prévention ou au traitement des maladies neurodégénératives centrales ou périphériques; de la sclérose latérale amyotrophique, de la sclérose en plaques; des affections cardiovasculaires; des neuropathies périphériques; des dommages au nerf optique et à la rétine; des traumatismes de la moëlle épinière et des traumatismes crâniens; de l'athérosclérose; des sténoses; de la cicatrisation; de l'alopécie; des cancers; des tumeurs ; des métastases ; des leucémies ; des douleurs chroniques neuropathiques et inflammatoires ; des maladies auto-immunes ; des fractures osseuses ; des maladies osseuses.

## Claims

1. Compound of the formula (I): 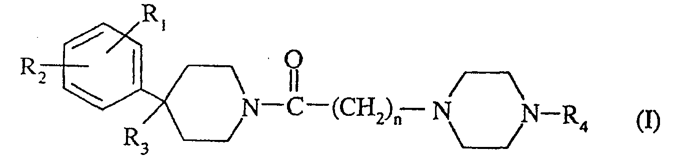 in which:
- n is 1 or 2;
- R₁ represents a halogen atom; a trifluoromethyl radical; a (C₁-C₄)alkyl; a (C₁-C₄)alkoxy; a trifluoromethoxy radical;
- R₂ represents a hydrogen atom or a halogen atom;
- R₃ represents a hydrogen atom; a group -OR₅; a group
- CH₂OR₅; a group -NR₆R₇; a group -NR₈COR₉; a group
- CH₂NR₁₀R_{11;} a (C₁-C₄)alkoxycarbonyl; a group -CONR₁₂R₁₃; a
- CN radical;
- R₄ represents a phenyl which is unsubstituted or mono-, di- or trisubstituted by a substituent selected independently from a halogen atom; a (C₁-C₄) alkyl a (C₁-C₄)alkoxy; a trifluoromethyl radical;
- R₅ represents a hydrogen atom or a (C₁-C₄)alkyl;
- R₆ and R₇ represent each independently a hydrogen atom or a (C₁-C₄)alkyl;
- R₈ represents a hydrogen atom or a (C₁-C₄)alkyl;
- R₉ represents a (C₁-C₄)alkyl;
- R₁₀ and R₁₁ represent each independently a hydrogen atom or a (C₁-C₄)alkyl; R₁₁ may also represent a group -CH₂R₁₄;
- or else R₁₀ and R₁₁, together with the nitrogen atom to which they are attached, constitute a heterocycle selected from aziridine, azetidine, pyrrolidine, piperidine and morpholine;
- R₁₂ and R₁₃ represent each independently a hydrogen atom or a (C₁-C₄)alkyl;
- or else R₁₂ and R₁₃, together with the nitrogen atom to which they are attached, constitute a heterocycle selected from azetidine, pyrrolidine, piperidine, morpholine and piperazine which is unsubstituted or substituted in position 4 by a (C₁-C₄)alkyl;
- R₁₄ represents an aromatic group selected from: 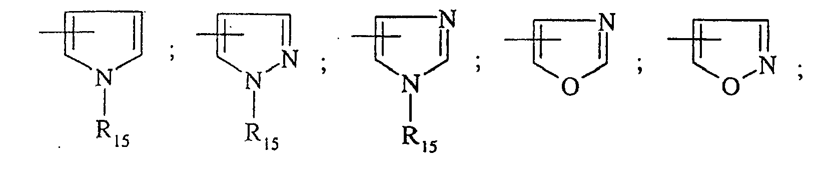 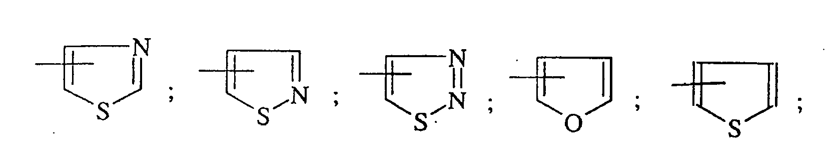 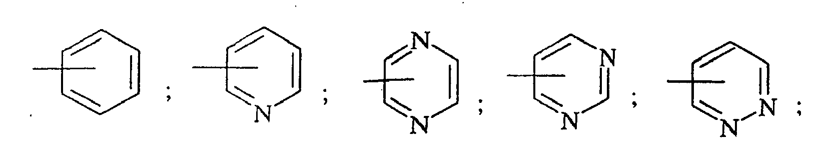 the said aromatic groups being unsubstituted, monosubstituted or disubstituted by a substituent selected independently from a halogen atom, a (C₁-C₃)alkyl or a (C₁-C₃)alkoxy;
- R₁₅ represents a hydrogen atom or a (C₁-C₃)alkyl;
in the form of a base or an addition salt with an acid, or in the form of a hydrate or solvate.

2. Compound of formula (I) according to Claim 1, **characterized in that**:
- n is 1 or 2;
in the form of a base or an addition salt with an acid, or in the form of a hydrate or solvate.

3. Compound of formula (I) according to Claim 1, **characterized in that**:
- R₁ is in position 3 of the phenyl and represents a trifluoromethyl radical;
in the form of a base or an addition salt with an acid, or in the form of a hydrate or solvate.

4. Compound of formula (I) according to Claim 1, **characterized in that**:
- R₂ represents a hydrogen atom;
in the form of a base or an addition salt with an acid, or in the form of a hydrate or solvate.

5. Compound of formula (I) according to Claim 1, **characterized in that**:
- R₃ represents a hydroxyl, an aminomethyl, a (2-furylmethylamino)methyl, a (2-thienylmethylamino)methyl, a (2-pyridylmethylamino)methyl, a (3-pyridylmethylamino)-methyl, a (4-pyridylmethylamino)methyl, a (methylamino)methyl, a (dimethylamino)methyl, an (N-methylethylamino)methyl, a (diethylamino)methyl, an azetidin-1-ylcarbonyl; an aminocarbonyl; an (N-methyl-2-furylmethylamino)methyl; a (3-furylmethylamino)methyl;
in the form of a base or an addition salt with an acid, or in the form of a hydrate or solvate.

6. Compound of formula (I) according to Claim 1, **characterized in that**:
- R₄ represents a phenyl, a 4-chlorophenyl, a 3-chlorophenyl, a 4-fluorophenyl, a 2,4-dichlorophenyl, a 3,4-dichlorophenyl, a 3,5-dichlorophenyl, a 4-methylphenyl, a 2,4-dimethylphenyl, a 3,4-dimethylphenyl, a 3-methoxyphenyl, a 4-methoxyphenyl, a 2,4-dimethoxyphenyl, a 3,4-dimethoxyphenyl, a 3-(trifluoromethyl)-phenyl; a 2,3-dimethylphenyl;
in the form of a base or an addition salt with an acid, and in the form of a hydrate or solvate.

7. Compound of formula (I) according to Claim 1, **characterized in that**:
- n is 1 or 2;
- R₁ is in position 3 of the phenyl and represents a trifluoromethyl radical;
- R₂ represents a hydrogen atom;
- R₃ represents a hydroxyl, an aminomethyl, a (2-furylmethylamino)methyl, a (2-thienylmethylamino)methyl, a (2-pyridylmethylamino)methyl, a (3-pyridylmethylamino)-methyl, a (4-pyridylmethylamino)methyl, a (methylamino)methyl, a (dimethylamino)methyl, an (N-methylethylamino)methyl, a (diethylamino)methyl, an azetidin-1-ylcarbonyl; an aminocarbonyl; an (N-methyl-2-furylmethylamino)methyl; a (3-furylmethylamino)methyl;
- R₄ represents a phenyl, a 4-chlorophenyl, a 3-chlorophenyl, a 4-fluorophenyl, a 2,4-dichlorophenyl, a 3,4-dichlorophenyl, a 3,5-dichlorophenyl, a 4-methylphenyl, a 2,4-dimethylphenyl, a 3,4-dimethylphenyl, a 3-methoxyphenyl, a 4-methoxyphenyl, a 2,4-dimethoxyphenyl, a 3,4-dimethoxyphenyl, a 3-(trifluoromethyl)-phenyl; a 2,3-dimethylphenyl;
in the form of a base or an addition salt with an acid, and in the form of a hydrate or solvate.

8. Process for preparing compounds of formula (I) according to Claim 1 in which n = 1, **characterized in that**:
a1) a compound of formula 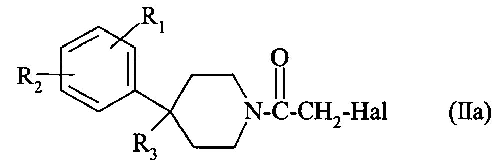 in which R₁, R₂ and R₃ are as defined for a compound of formula (I) in Claim 1 and Hal represents a halogen atom, preferably chlorine or bromine, with the proviso that when R₃ contains a hydroxyl or amine function these functions may be protected is reacted with a compound of formula 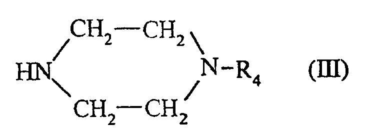 in which R₄ is as defined for a compound of formula (I) in Claim 1;
b1) and, after deprotection of the hydroxyl or amine functions present in R₃ where appropriate, the compound of formula (I) is obtained.

9. Process for preparing compounds of formula (I) according to Claim 1 in which n = 2, **characterized in that**:
a2) a compound of formula 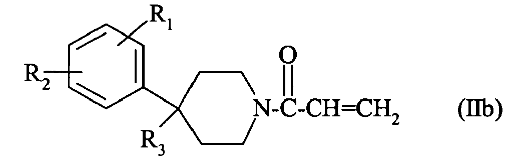 in which R₁, R₂ and R₃ are as defined for a compound of formula (I) in Claim 1, with the proviso that when R₃ contains a hydroxyl or amine function these functions may be protected, is reacted with a compound of formula 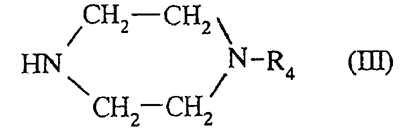 in which R₄ is as defined for a compound of formula (I) in Claim 1;
b2) and, after deprotection of the hydroxyl or amine functions present in R₃ where appropriate, the compound of formula (I) is obtained.

10. Process for preparing compounds of formula (I) according to Claim 1 in which R₃ represents a group - CH₂NR₁₀R₁₁ in which R₁₀ and R₁₁ each represent hydrogen, **characterized in that**:
a3) a compound of formula 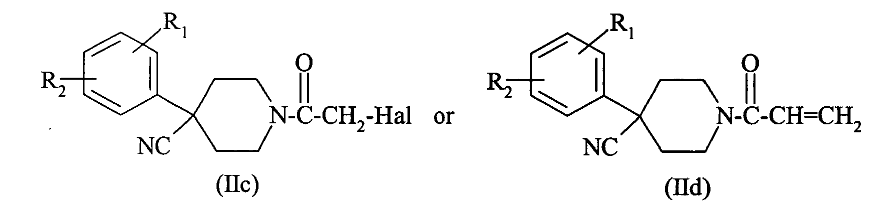 in which R₁ and R₂ are as defined for a compound of formula (I) in Claim 1 and Hal represents a halogen atom, preferably chlorine or bromine, is reacted with a compound of formula 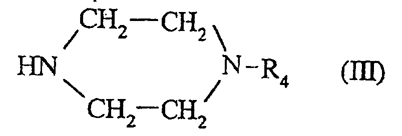 in which R₄ is as defined for a compound of formula (I) in Claim 1 to give a compound of formula 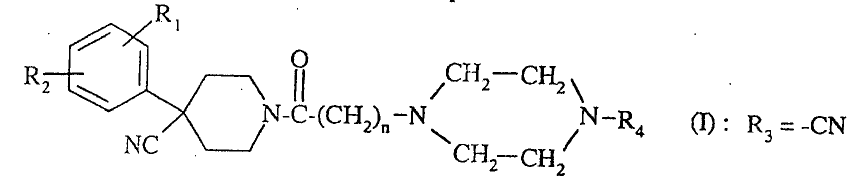
b3) the cyano group of the compound of formula (I) is reduced to give a compound of formula (I) according to Claim 1 in which R₃ = CH₂NH₂.

11. Process for preparing compounds of formula (I) according to Claim 1 in which R₃ represents a group - CN₂NR₁₀R₁₁ in which R₁₁ represents a group -CH₂R₁₄:
a4) a compound of formula: 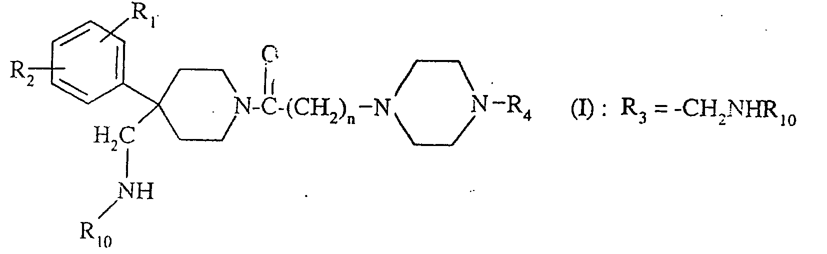 in which R₁, R₂, R₄, R₁₀ and n are as defined for a compound of formula (I) in Claim 1 is reacted with a compound of formula: 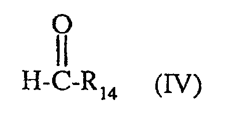 in which R₁₄ is as defined for a compound of formula (I) in Claim 1, in the presence of an acid, in a solvent, and then the iminium salt intermediate formed is reduced by means of a reducing agent.

12. Medicament, **characterized in that** it comprises a compound of formula (I) according to any one of Claims 1 to 7, or an addition salt of this compound with a pharmaceutically acceptable acid, or else a hydrate or a solvate of the compound of formula (I).

13. Pharmaceutical composition, **characterized in that** it comprises a compound of formula (I) according to any one of Claims 1 to 7, or a pharmaceutically acceptable salt, a hydrate or a solvate of this compound, and at least one pharmaceutically acceptable excipient.

14. Use of a compound of formula (I) according to any one of Claims 1 to 7 for the preparation of a medicament intended for the prevention or treatment of central or peripheral neurodegenerative diseases; amyotrophic lateral sclerosis, multiple sclerosis; cardiovascular conditions; peripheral neuropathies; damage to the optic nerve and to the retina; spinal cord trauma and cranial trauma; atherosclerosis; stenoses; cicatrization; alopecia; cancers; tumours; metastases; leukaemias; chronic neuropathic and inflammatory pain; autoimmune diseases; bone fractures; bone diseases.

## Patentansprüche

1. Verbindung der Formel (I): 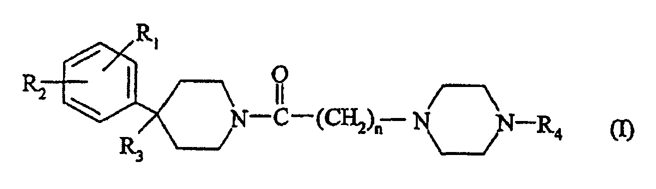 worin:
- n für 1 oder 2 steht;
- R₁ für ein Halogenatom; einen Trifluormethylrest;
(C₁-C₄)-Alkyl; (C₁-C₄)-Alkoxy oder einen Trifluormethoxyrest steht;
- R₂ für ein Wasserstoffatom oder ein Halogenatom steht;
- R₃ für ein Wasserstoffatom; eine Gruppe -OR₅; eine Gruppe -CH₂OR₅; eine Gruppe -NR₆R_{7;} eine Gruppe -NR₈COR₉; eine Gruppe -CH₂NR₁₀R₁₁; (C₁-C₄) -Alkoxycarbonyl; eine Gruppe -CONR₁₂R₁₃ oder einen -CN-Rest steht;
- R₄ für Phenyl steht, das gegebenenfalls ein-, zwei- oder dreifach durch einen unabhängig unter einem Halogenatom, (C₁-C₄)-Alkyl, (C₁-C₄)-Alkoxy und einem Trifluormethylrest ausgewählten Substituenten substituiert ist;
- R₅ für ein Wasserstoffatom oder (C₁-C₄)-Alkyl steht;
- R₆ und R₇ jeweils unabhängig voneinander für ein Wasserstoffatom oder (C₁-C₄)-Alkyl stehen;
- R₈ für ein Wasserstoffatom oder (C₁-C₄)-Alkyl steht;
- R₉ für (C₁-C₄)-Alkyl steht;
- R₁₀ und R₁₁ jeweils unabhängig voneinander für ein Wasserstoffatom oder (C₁-C₄)-Alkyl stehen; R₁₁ außerdem für eine Gruppe -CH₂R₁₄ stehen kann;
- oder R₁₀ und R₁₁ zusammen mit dem Stickstoffatom, an das sie gebunden sind, einen unter Aziridin, Azetidin, Pyrrolidin, Piperidin und Morpholin ausgewählten Heterocyclus bilden;
- R₁₂ und R₁₃ jeweils unabhängig voneinander für ein Wasserstoffatom oder (C₁-C₄)-Alkyl stehen;
- oder R₁₂ und R₁₃ zusammen mit dem Stickstoffatom, an das sie gebunden sind, einen unter Azetidin, Pyrrolidin, Piperidin, Morpholin oder Piperazin, das gegebenenfalls in 4-Position durch (C₁-C₄) - Alkyl substituiert ist, ausgewählten Heterocyclus bilden;
- R₁₄ für eine unter: 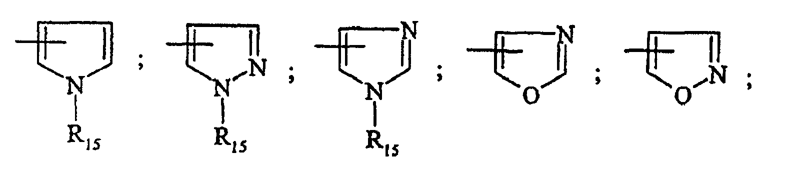 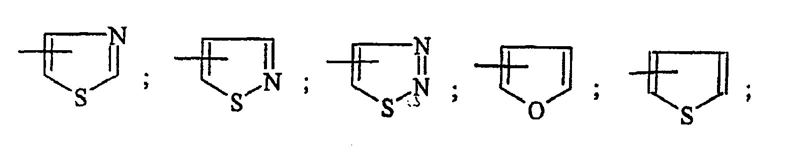 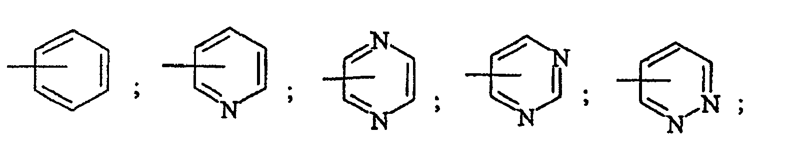 ausgewählte aromatische Gruppe steht, wobei die aromatischen Gruppen gegebenenfalls ein- oder zweifach durch einen unabhängig unter einem Halogenatom, (C₁-C₃) -Alkyl und (C₁-C₃) -Alkoxy ausgewählten Substituenten substituiert sind;
- R₁₅ für ein Wasserstoffatom oder (C₁-C₃)-Alkyl steht;
in Basen- oder Säureadditionssalzform sowie in Hydrat- oder Solvatform.

2. Verbindung der Formel (I) nach Anspruch 1, **dadurch gekennzeichnet, daß**:
- n für 1 oder 2 steht;
in Basen- oder Säureadditionssalzform sowie in Hydrat- oder Solvatform.

3. Verbindung der Formel (I) nach Anspruch 1, **dadurch gekennzeichnet, daß**:
- R₁ in 3-Position des Phenyls steht und für einen Trifluormethylrest steht;
in Basen- oder Säureadditionssalzform sowie in Hydrat- oder Solvatform.

4. Verbindung der Formel (I) nach Anspruch 1, **dadurch gekennzeichnet, daß**:
- R₂ für ein Wasserstoffatom steht;
in Basen- oder Säureadditionssalzform sowie in Hydrat- oder Solvatform.

5. Verbindung der Formel (I) nach Anspruch 1, **dadurch gekennzeichnet, daß**:
- R₃ für Hydroxy, Aminomethyl, (2-Furylmethylamino)methyl, (2-Thienylmethylamino)methyl, (2-Pyridinylmethylamino)methyl, (3-Pyridinylmethylamino)methyl, (4-Pyridinylmethylamino)methyl, (Methylamino)methyl, (Dimethylamino)methyl, (N-Methylethylamino)methyl, (Diethylamino)methyl, Azetidin-1-ylcarbonyl; Aminocarbonyl; (N-Methyl-2-furylmethylamino)methyl oder (3-Furylmethylamino)-methyl steht;
in Basen- oder Säureadditionssalzform sowie in Hydrat- oder Solvatform.

6. Verbindung der Formel (I) nach Anspruch 1, **dadurch gekennzeichnet, daß**:
- R₄ für Phenyl, 4-Chlorphenyl, 3-Chlorphenyl, 4-Fluorphenyl, 2,4-Dichlorphenyl, 3,4-Dichlorphenyl, 3,5-Dichlorphenyl, 4-Methylphenyl, 2,4-Dimethylphenyl, 3,4-Dimethylphenyl, 3-Methoxyphenyl, 4-Methoxyphenyl, 2,4-Dimethoxyphenyl, 3,4-Dimethoxyphenyl, 3-(Trifluormethyl)phenyl oder 2,3-Dimethylphenyl steht;
in Basen- oder Säureadditionssalzform sowie in Hydrat- oder Solvatform.

7. Verbindung der Formel (I) nach Anspruch 1, **dadurch gekennzeichnet, daß**:
- n für 1 oder 2 steht;
- R₁ in 3-Position des Phenyls steht und für einen Trifluormethylrest steht;
- R₂ für ein Wasserstoffatom steht;
- R₃ für Hydroxy, Aminomethyl, (2-Furylmethylamino)methyl, (2-Thienylmethylamino)methyl, (2-Pyridinylmethylamino)methyl, (3-Pyridinylmethylamino)methyl, (4-Pyridinylmethylamino)methyl, (Methylamino)methyl, (Dimethylamino)methyl, (N-Methylethylamino)methyl, (Diethylamino)methyl, Azetidin-1-ylcarbonyl; Aminocarbonyl; (N-Methyl-2-furylmethylamino)methyl oder (3-Furylmethylamino)-methyl steht;
- R₄ für Phenyl, 4-Chlorphenyl, 3-Chlorphenyl, 4-Fluorphenyl, 2,4-Dichlorphenyl, 3,4-Dichlorphenyl, 3,5-Dichlorphenyl, 4-Methylphenyl, 2,4-Dimethylphenyl, 3,4-Dimethylphenyl, 3-Methoxyphenyl, 4-Methoxyphenyl, 2,4-Dimethoxyphenyl, 3,4-Dimethoxyphenyl, 3-(Trifluormethyl)phenyl oder 2,3-Dimethylphenyl steht;
in Basen- oder Säureadditionssalzform sowie in Hydrat- oder Solvatform.

8. Verfahren zur Herstellung von Verbindungen der Formel (I) nach Anspruch 1 mit n = 1, **dadurch gekennzeichnet, daß** man:
a1) eine Verbindung der Formel: 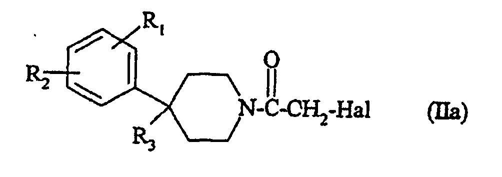 worin R₁, R₂ und R₃ die für eine Verbindung der Formel (I) in Anspruch 1 angegebene Bedeutung besitzen und Hal für ein Halogenatom, vorzugsweise Chlor oder Brom, steht, mit der Maßgabe, daß dann, wenn R₃ eine Hydroxyl- oder Aminfunktion enthält, diese Funktionen geschützt sein können, mit einer Verbindung der Formel: 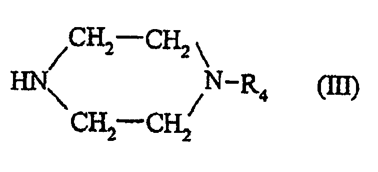 worin R₄ die für eine Verbindung der Formel (I) in Anspruch 1 angegebene Bedeutung besitzt, umsetzt b1) und nach eventueller Entschützung der in R₃ enthaltenen Hydroxyl- oder Aminfunktionen die Verbindung der Formel (I) erhält.

9. Verfahren zur Herstellung von Verbindungen der Formel (I) nach Anspruch 1 mit n = 2, **dadurch gekennzeichnet, daß** man:
a2) eine Verbindung der Formel: 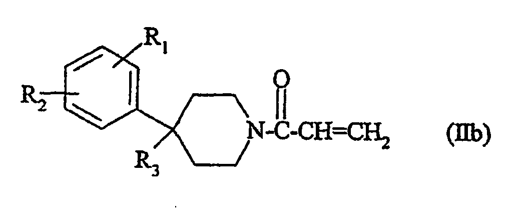 worin R₁, R₂ und R₃ die für eine Verbindung der Formel (I) in Anspruch 1 angegebene Bedeutung besitzen, mit der Maßgabe, daß dann, wenn R₃ eine Hydroxyl- oder Aminfunktion enthält, diese Funktionen geschützt sein können, mit einer Verbindung der Formel: 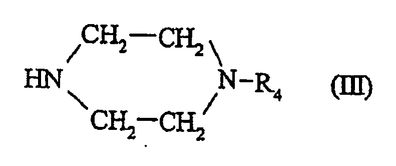 worin R₄ die für eine Verbindung der Formel (I) in Anspruch 1 angegebene Bedeutung besitzt, umsetzt b2) und nach eventueller Entschützung der in R₃ enthaltenen Hydroxyl- oder Aminfunktionen die Verbindung der Formel (I) erhält.

10. Verfahren zur Herstellung von Verbindungen der Formel (I) nach Anspruch 1, worin R₃ für eine Gruppe -CH₂NR₁₀R₁₁, worin R₁₀ und R₁₁ jeweils Wasserstoff bedeuten, steht, **dadurch gekennzeichnet, daß** man:
a3) eine Verbindung der Formel: 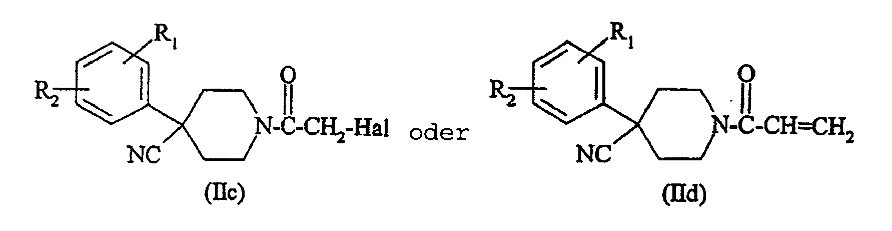 worin R₁ und R₂ die für eine Verbindung der Formel (I) in Anspruch 1 angegebene Bedeutung besitzen und Hal für ein Halogenatom, vorzugsweise Chlor oder Brom, steht, mit einer Verbindung der Formel: 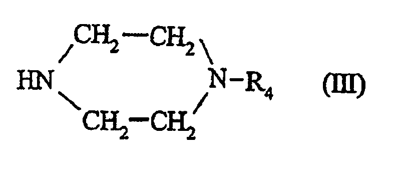 worin R₄ die für eine Verbindung der Formel (I) in Anspruch 1 angegebene Bedeutung besitzt, zu einer Verbindung der Formel: 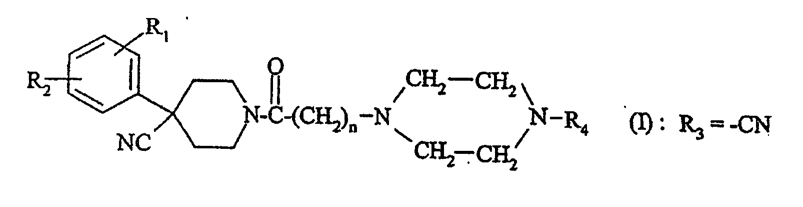 umsetzt;
b3) die Cyanogruppe der Verbindung der Formel (I) reduziert, wobei man eine Verbindung der Formel (I) nach Anspruch 1 mit R₃ = CH₂NH₂ erhält.

11. Verfahren zur Herstellung von Verbindungen der Formel (I) nach Anspruch 1, worin R₃ für eine Gruppe -CH₂NR₁₀R₁₁, worin R₁₁ eine Gruppe -CH₂R₁₄ bedeutet, steht, bei dem man:
a4) eine Verbindung der Formel: 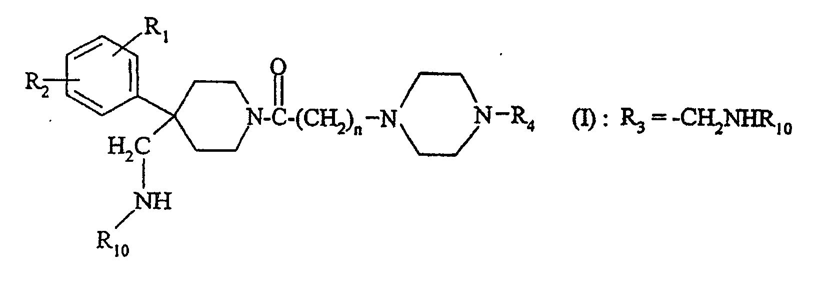 worin R₁, R₂, R₄, R₁₀ und n die für eine Verbindung der Formel (I) in Anspruch 1 angegebene Bedeutung besitzen, in Gegenwart einer Säure in einem Lösungsmittel mit einer Verbindung der Formel: 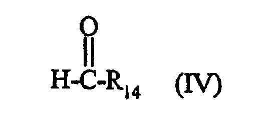 worin R₁₄ die für eine Verbindung der Formel (I) in Anspruch 1 angegebene Bedeutung besitzt, umsetzt und dann das intermediär gebildete Iminiumsalz mit Hilfe eines Reduktionsmittels reduziert.

12. Arzneimittel, **dadurch gekennzeichnet, daß** es eine Verbindung der Formel (I) nach einem der Ansprüche 1 bis 7 oder ein Additionssalz dieser Verbindung mit einer pharmazeutisch unbedenklichen Säure oder auch ein Hydrat oder ein Solvat der Verbindung der Formel (I) enthält.

13. Pharmazeutische Zusammensetzung, **dadurch gekennzeichnet, daß** sie eine Verbindung der Formel (I) nach einem der Ansprüche 1 bis 7 oder ein pharmazeutisch unbedenkliches Salz, ein Hydrat oder ein Solvat dieser Verbindung sowie mindestens einen pharmazeutisch unbedenklichen Hilfsstoff enthält.

14. Verwendung einer Verbindung der Formel (I) nach einem der Ansprüche 1 bis 7 zur Herstellung eines Arzneimittels zur Prävention oder Behandlung von von zentralen oder peripheren neurodegenerativen Erkrankungen; amyotropher Lateralsklerose, multipler Sklerose; Herz-Kreislauf-Leiden; peripheren Neuropathien; Schäden des Sehnervs und der Retina; Rückenmarks- und Schädeltraumata; Atherosklerose; Stenosen; Vernarbung; Alopecia; Krebserkrankungen; Tumoren; Metastasen; Leukämien; chronischen neuropathischen und entzündlichen Schmerzen; Autoimmunerkrankungen; Knochenbrüchen und Knochenerkrankungen.
